# EUROPEAN PATENT SPECIFICATION

(11) **EP 4 107 529 B1**
(45) Date of publication and mention of the grant of the patent: **13.11.2024**
(21) Application number: 21705980.7
(22) Date of filing: 19.02.2021
(51) Int. Cl.: G01N 33/68

(54) **METHODS FOR THE QUANTIFICATION OF GLYCOPROTEINS**
VERFAHREN ZUR QUANTIFIZIERUNG VON GLYCOPROTEINEN
PROCÉDÉS POUR LA QUANTIFICATION DE GLYCOPROTÉINES

(30) Priority: 21.02.2020 EP 20158905
(43) Date of publication of application: 28.12.2022
(73) Proprietor: Genmab B.V., 3584 CT Utrecht (NL)
(72) Inventor: VAN DEN BREMER, Ewald, T.J., 3584 CT Utrecht (NL); SANGERS, Sipke, 3584 CT Utrecht (NL); WILLEMSZ, Demelza, 3584 CT Utrecht (NL); HIBBERT, Richard, G., 3584 CT Utrecht (NL); DE JONG, Rob, N., 3584 CT Utrecht (NL); JENSEN, Søren, Skov, 1920 Frederiksberg C (DK); POULSEN, Emil, 2500 Valby (DK)
(74) Representative: Genmab A/S
(86) International application number: PCT/EP2021/054194
(87) International publication number: WO 2021/165488

(56) References cited:
- KALYAN RAO ANUMULA ED - SANCHEZ ANA ET AL: "Quantitative glycan profiling of normal human plasma derived immunoglobulin and its fragments Fab and Fc", JOURNAL OF IMMUNOLOGICAL METHODS, ELSEVIER SCIENCE PUBLISHERS B.V.,AMSTERDAM, NL, vol. 382, no. 1, 30 May 2012 (2012-05-30), pages 167 - 176, XP028399735, ISSN: 0022-1759, [retrieved on 20120606], DOI: 10.1016/J.JIM.2012.05.022
- S. HUNTER WALKER ET AL: "The use of a xylosylated plant glycoprotein as an internal standard accounting for N -linked glycan cleavage and sample preparation variability", RAPID COMMUNICATIONS IN MASS SPECTROMETRY., vol. 27, no. 12, 7 May 2013 (2013-05-07), GB, pages 1354 - 1358, XP055713606, ISSN: 0951-4198, DOI: 10.1002/rcm.6579
- S. HUNTER WALKER ET AL: "Stable-Isotope Labeled Hydrophobic Hydrazide Reagents for the Relative Quantification of N-Linked Glycans by Electrospray Ionization Mass Spectrometry", ANALYTICAL CHEMISTRY, vol. 83, no. 17, 20 July 2011 (2011-07-20), US, pages 6738 - 6745, XP055713612, ISSN: 0003-2700, DOI: 10.1021/ac201376q
- YINING HUANG ET AL: "Kinetics of N-Glycan Release from Human Immunoglobulin G (IgG) by PNGase F: All Glycans Are Not Created Equal", JOURNAL OF BIOMOLECULAR TECHNIQUES, vol. 28, no. 4, 1 December 2017 (2017-12-01), US, pages 150 - 157, XP055713889, ISSN: 1524-0215, DOI: 10.7171/jbt.17-2804-002
- "Methods in Molecular Biology", vol. 1827, 1 January 2018, HUMANA PRESS, NEW YORK, NY, US, ISSN: 1064-3745, article CHAO LI ET AL: "Chemoenzymatic Defucosylation of Therapeutic Antibodies for Enhanced Effector Functions Using Bacterial [alpha]-Fucosidases : Methods and Protocols", pages: 367 - 380, XP055714339, DOI: 10.1007/978-1-4939-8648-4_19
- TIEZHENG LI ET AL: "Modulating IgG effector function by Fc glycan engineering", PROCEEDINGS OF THE NATIONAL ACADEMY OF SCIENCES, vol. 114, no. 13, 13 March 2017 (2017-03-13), US, pages 3485 - 3490, XP055428860, ISSN: 0027-8424, DOI: 10.1073/pnas.1702173114

## Description

### FIELD OF THE INVENTION

The present invention relates to methods for the quantification of glycoproteins in a sample and to materials for use in such methods.

### BACKGROUND OF THE INVENTION

Recombinant proteins are increasingly being developed for use e.g. as drug products. Analytical tools such as liquid chromatography (LC) and capillary electrophoresis (CE) have been successfully used to separate and quantify size and charged variants of intact monoclonal antibody variants (Staub et al, J Pharm Biomed Anal 55, 810-822, 2011). In addition, immunoassays such as the enzyme-linked immunosorbent assay (ELISA) were implemented for quantitation purposes as well, making use of specific antibodies against target proteins e. g. in human serum or plasma (Mushens et al, J. Immunol. Methods, 162 (1), 1993). LC-mass spectrometry (MS) is a commonly used method for protein analysis and quantification. In 'bottom-up' MS approaches enzymatic digestion of a target protein produces a signature peptide that is subsequently quantified in the presence of a stable-isotope-labeled (SIL) peptide as Internal Standard (IS) (US20120264155A1). The increasing interest for intact protein quantitation is driven by some drawbacks in peptide level quantitation (Jin et al, Bioanalysis 10(11), 851-862, 2018). Selection of the most appropriate signature peptides and the optimization of the enzymatic digestion steps make assay development complex and time-consuming. In addition, enzymatic protein digestion increases the spectral complexity making the analysis more susceptible to interferences. However, the major limitation in peptide level quantitation is that information about the whole protein molecule is lost upon digestion and any given peptide may not truly represent the whole protein. Macchi et al. describe an MS-based method to quantitate intact bispecific antibodies and variants thereof (Macchi et al., Anal Chem 87:10475-82 (2015)). However, LC-MS based quantitation of intact antibody mixtures provides challenges related to size, structural complexity and heterogeneity in both LC and MS. Hence, the use of intact surrogate protein species as IS remains limited.

Immunoglobulins are glycoproteins. IgG1, IgG2 and IgG4 have a single conserved Asn-linked glycosylation site in the CH2 region (at position Asn297 for IgG1) and thus carry two glycans per immunoglobulin molecule. Other immunoglobulins are more heavily glycosylated (see e.g. Maverakis et al. (2015) J Autoimmun, 57: 1-13). The IgG-Fc Asn-linked glycan is a highly heterogeneous post-translational modification due to the variable addition of fucose, galactose, and sialic acid residues to the core complex biantennary heptasaccharide (GlcNAc2Man3GlcNAc2). Glycans play an important role in protein conformation, stability and biological function (Costa et al., Crit Rev Biotechnol 34(4): 281-99 (2014)). The heterogeneity of Fc glycans varies with species and expression system. Mammalian cell lines are commonly applied to manufacture recombinant glycoproteins, with Chinese hamster ovary (CHO)-based expression systems used most commonly. Recombinant biotherapeutics are also produced in other expression systems, such as yeast, plants or insects (Lalonde et al., J Biotechnol 251:128-40 (2017)). However, these latter organisms produce glycan structures that vary from those produced in mammalian cells due to differences in the enzymatic machinery.

Deglycosylation of glycoprotein samples can be accomplished by a variety of glycosidases, such as Peptide:N-glycosidase F (PNGase F), with each enzyme having its unique glycan cleavage pattern (Seki et al., (2019) J. Biol. Chem. 294(45): 17143-54). LC-MS analysis following deglycosylation has been applied to characterize the N-glyco-occupancy of therapeutic monoclonal antibodies (Liu et al, Anal Biochem 509 p142-45, 2016).

K. R. Anumulada, Journal of Immunological Methods, Vol. 382, No. 1, 2012, pp167-176, Walker et al., Rapid Communications in Mass Spectrometry, Vol. 27, No. 12, 2013, pp. 1354-1358 and Walker et al., Analytic Chemistry, Vol. 83, No. 17, 2011, pp. 6738-6745 all disclose the use of a labeled GO or GOF core glycoform as an internal standard for the quantification of N-linked glycan structures on proteins in a sample.

Chao et al., Methods in Molecular Biology, 1 January 2018, Humana Press New York, NY US, vol. 1827, pp. 367-380 and Tiezheng et al., Proc. Natl. Acad. Sci USA, vol. 114, no. 13, 1997, pp. 3485-3490 each disclose antibodies, which have been treated with EndoS2 followed by alpha-L-fucosidase. The resulting antibody has N-linked structures consisting of one Asn-linked GlcNac residue.

### SUMMARY OF THE INVENTION

There remains a need for improved methods to accurately quantify proteins in samples, in particular within protein mixtures.

The present invention provides a method for the quantification of glycoproteins which uses an internal standard that has biophysical properties that are highly comparable to the individual protein sample components in a mixture, thereby enabling accurate quantification of the composition of such protein samples.

The internal standard used in the method of the invention is a variant form of the glycoprotein(s) to be quantified which only contains core GicNAc moieties rather than a full glycan structure. The method of the invention exploits the resulting difference in mass between the glycoprotein in the sample and the variant form of the internal standard for quantification of the protein in the sample relative to the internal standard.

Accordingly, in a first aspect, the invention relates to a method for the quantification of one or more glycoproteins in a sample, wherein each glycoprotein to be quantified comprises one or more Asn-linked glycans, said method comprising the steps of:
a. providing a sample comprising said one or more glycoproteins to be quantified,
b. adding an internal standard to said sample, wherein said internal standard comprises a variant form of each of said one or more glycoproteins to be quantified, wherein said variant form is a form containing only core Asn-linked GlcNAc moieties, each core Asn-linked GlcNAc moiety being an N-acetylglycosamine moiety that is directly attached to an Asn acceptor residue in the polypeptide chain of said variant, and wherein no fucose moieties are linked to said core GlcNAc moieties, and
c. quantifying said one or more glycoproteins by comparison with the internal standard.

In a further aspect, the invention relates to a method for the quantification of one or more glycoproteins in a sample, wherein each glycoprotein to be quantified comprises one or more Asn-linked glycans, said method comprising the steps of:
a. preparing an internal standard by treating said one or more glycoproteins with one or more enzymes to obtain a variant form of each of said one or more glycoproteins, wherein said variant form is a form containing only core Asn-linked GlcNAc moieties, each core Asn-linked GlcNAc moiety being an N-acetylglycosamine moiety that is directly attached to an Asn acceptor residue in the polypeptide chain of said variant, and wherein no fucose moieties are linked to said core GlcNAc moieties,
b. providing a sample comprising said one or more glycoproteins to be quantified,
c. adding said internal standard to said sample, and
d. quantifying said one or more glycoproteins by comparison with the internal standard.

### BRIEF DESCRIPTION OF THE DRAWINGS

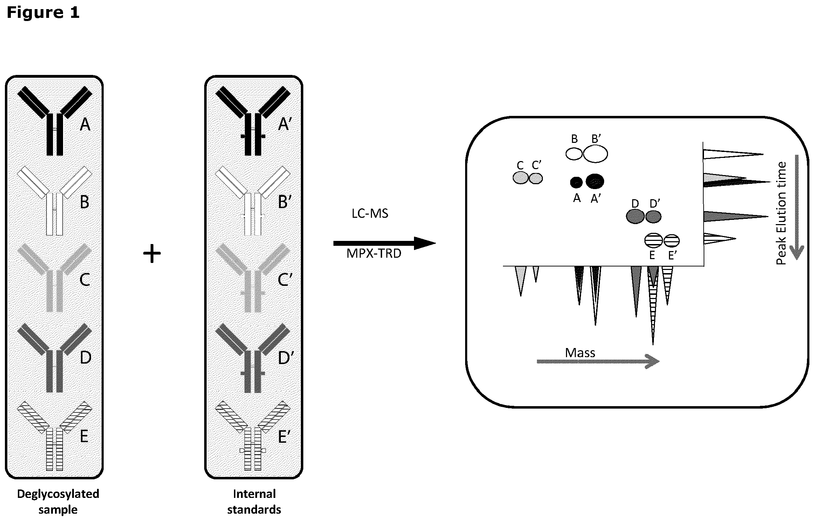

**Figure 1** shows a schematic overview of a potential application of the methods described. In this example, a mixture of 5 IgG antibodies could be quantitated using 5 variant antibodies that serve as internal standards for the 5 antibodies to be quantitated. These internal standards are identical to the fully deglycosylated antibodies to be quantitated with the exception of a residual core GlcNAc moiety on the internal standards to distinguish them from the antibodies to be quantitated. By applying Time-Resolved Deconvolution (TRD) in which the observed masses (x-axis) are plotted against elution time (y-axis), a two-dimensional view could be generated allowing the identification of the individual antibody samples and internal standards.
In the third dimension, peak intensities are preserved in such a way that peak volumes can be deduced for further quantitative calculations.

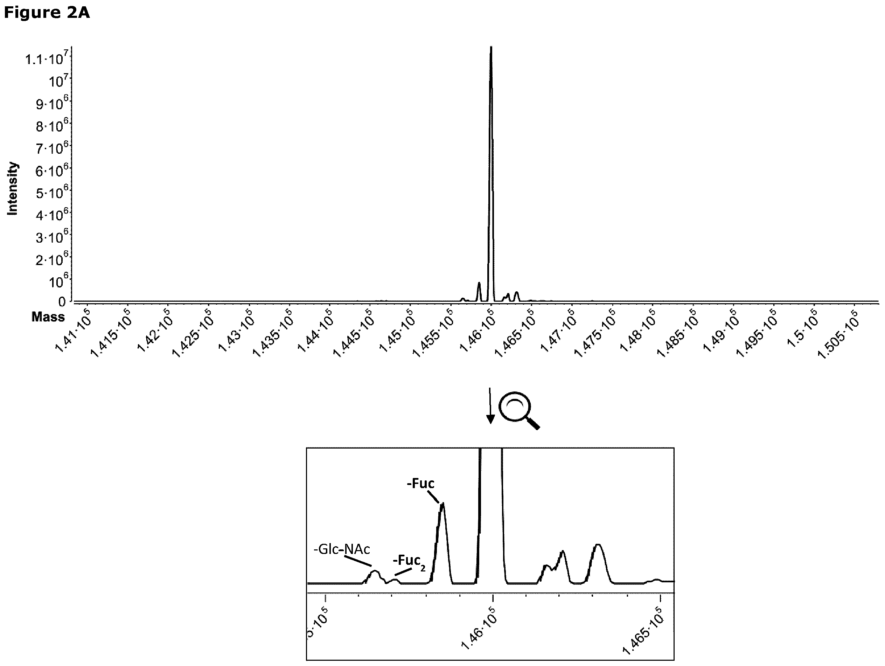

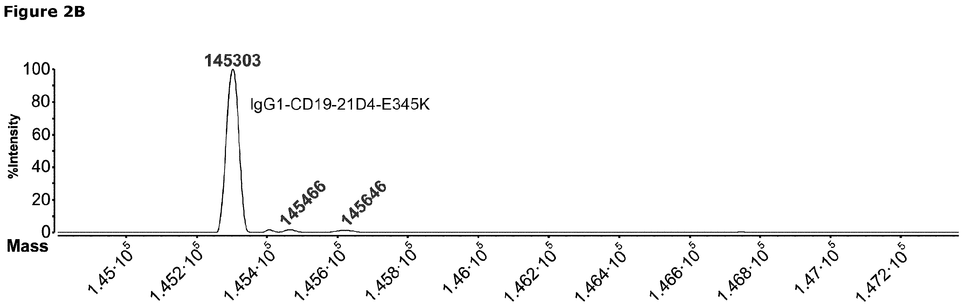

**Figure 2** shows that enzymatic deglycosylation of IgG antibodies by EndoS2 results in sample heterogeneity caused by the presence of afucosylated species. (A) Enzymatic deglycosylation of IgG1-CD19-21D4-E345K with EndoS2 results in efficient removal of the glycans up to the core Asn-linked GlcNAc group. However, the presence of afucosylated IgG1-CD19-21D4-E345K results in sample heterogeneity, with some antibodies containing two heavy chains with a GlcNAc-fucose moiety, some containing one heavy chain with a GlcNAc-fucose moiety and one heavy chain with a GlcNAc moiety without fucose attached thereto, and some containing two heavy chains with GlcNAc moieties without fucose attached thereto. (B) Enzymatic deglycosylation of IgG1-CD19-21D4-E345K with PNGase F results in a homogeneous population of fully deglycosylated IgG1-CD19-21D4-E345K.

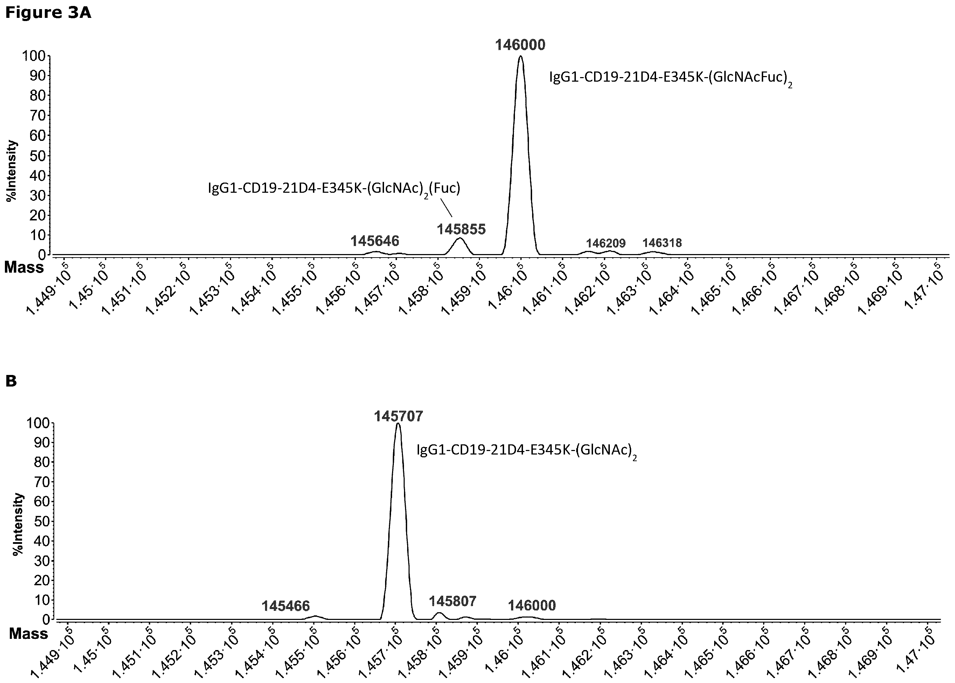

**Figure 3** shows that enzymatic treatment of EndoS2-treated IgG antibodies with o-L-fucosidase from *L. casei* efficiently removed the residual fucose groups, resulting in decreased mass heterogeneity. In panel A, IgG1-CD19-21D4-E345K was treated with EndoS2 only, resulting in a large fraction of IgG-(GlcNAc-Fuc)₂ and some IgG-(GlcNAc-Fuc), and IgG-(GlcNAc). In panel B, IgG1-CD19-21D4-E345K was treated with both EndoS2 and o-L-fucosidase, resulting in a single peak of IgG-(GlcNAc).

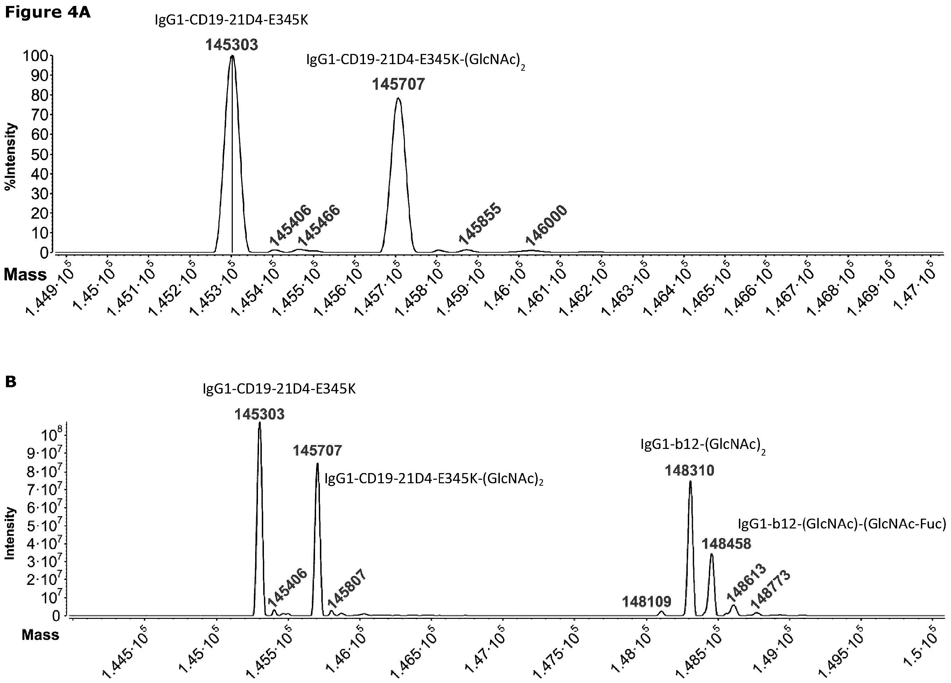

**Figure 4** shows that PNGase F from PNGase F-treated samples does not remove the residual GlcNAc moiety from IgG antibodies treated with EndoS2 and o-L-fucosidase. (A) PNGase F from PNGase F-treated sample IgG1-CD19-21D4-E345K does not act on the EndoS2- and o-L-fucosidase-treated internal standard IgG1-CD19-21D4-E345K-(GlcNAc)₂. (B) PNGase F from PNGase F-treated sample IgG1-CD19-21D4-E345K does not act on the EndoS2- and o-L-fucosidase-treated internal standard IgG1-CD19-21D4-E345K-(GlcNAc)₂ or on untreated IgG1-b12, while EndoS2- and o-L-fucosidase from the internal standard do act on untreated IgG1-b12.

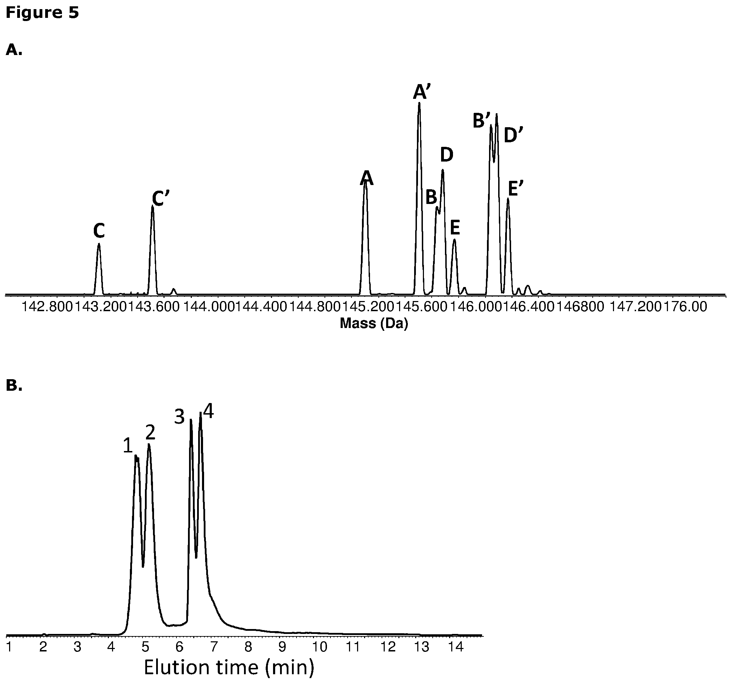

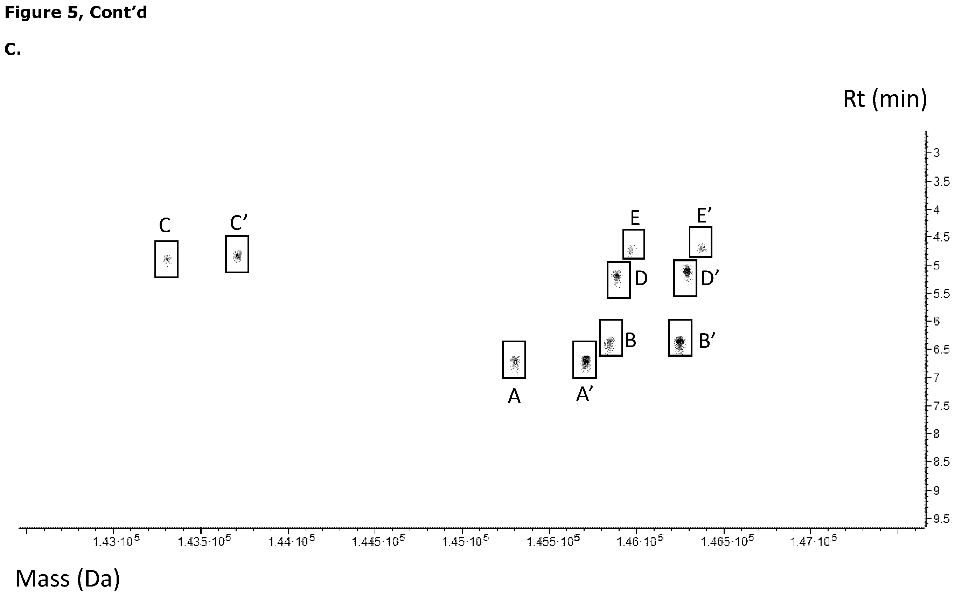

**Figure 5** shows MS analyses and LC analyses of an antibody mixture composed of deglycosylated human IgG1-CD19-21D4-E345K, IgG1-CD22-huRFB4, IgG1-7D8, IgG1-CD37-37-3 and IgG1-CD52-Campath-E345K antibodies, and the corresponding internal standards of these antibodies harboring an Asn-linked GlcNAc moiety on each heavy chain. (A) MS spectrum indicating overlapping antibody mass peaks and matching internal standards processed by conventional deconvolution mass spectrometry. (B) Elution time of the antibodies and variant antibody internal standards mentioned in (A) as determined through LC analysis. (C) Time-resolved deconvolution mass spectrum of MS and LC data for the antibody sample mixture and variant internal standards mentioned in (A), with the mass (Da) of each sample mixture or standard mixture on the x-axis and the elution time (min) on the y-axis. Squares indicate the boundaries for peak volume integration.

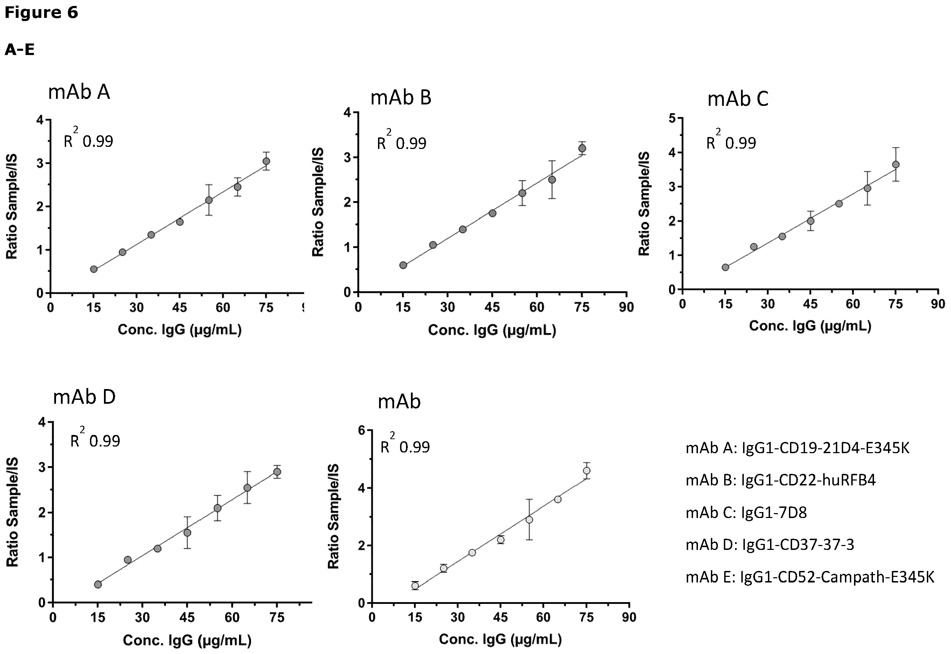

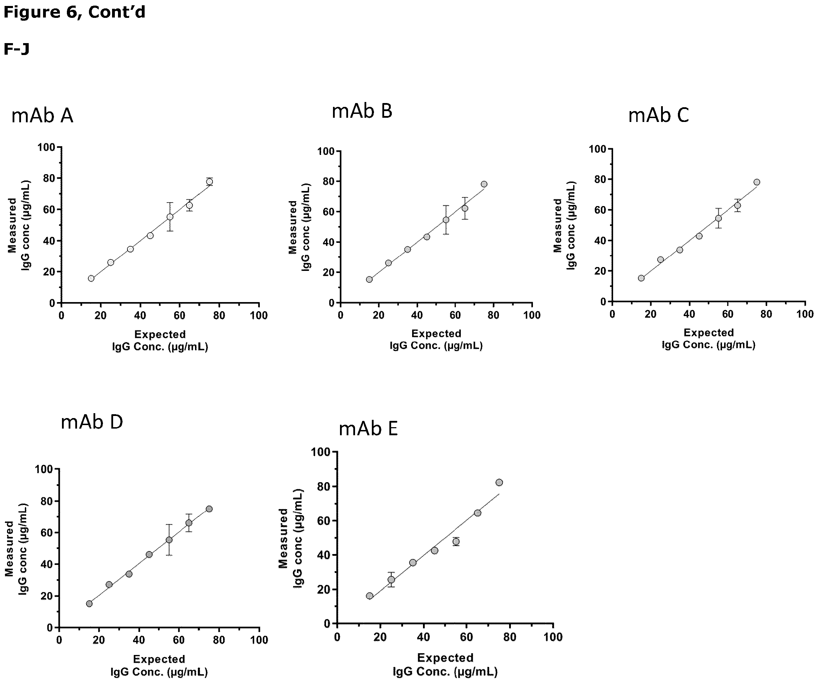

**Figure 6** shows the Sample/IS ratios plotted against the known antibody concentrations (µg/mL) for each antibody tested. Results are shown for (A) IgG1-CD19-21D4-E345K, (B) IgG1-CD22-huRFB4, (C) IgG1-7D8, (D) IgG1-CD37-37-3 and (E) IgG1-CD52-Campath-E345K. Figures 6F-J show the antibody concentrations (µg/mL) determined using time-resolved deconvolution MS, as plotted versus the expected, known antibody concentrations (µg/mL). Results are shown for (F) IgG1-CD19-21D4-E345K, (G) IgG1-CD22-huRFB4, (H) IgG1-7D8, (I) IgG1-CD37-37-3 and (J) IgG1-CD52-Campath-E345K.

### DETAILED DESCRIPTION OF THE INVENTION

### Definitions

The term "glycoprotein" when used herein refers to a protein which contains one or more glycans (i.e. oligosaccharide- or carbohydrate- side chains) covalently attached to the polypeptide chain. The process of attaching glycans to a polypeptide is known as glycosylation. One of the most common types of glycosylation is Asn-linked glycosylation, wherein a glycan is attached to the amide nitrogen of an asparagine residue of a polypeptide.

The term "internal standard" has its usual meaning in the art and refers to a known quantity of a substance, herein a variant glycoprotein, that is added to samples containing an analyte, e.g. a glycoprotein, to be quantified. This substance can then be used for quantification of the analyte by determining the ratio of the analyte signal to the internal standard signal.

The term "immunoglobulin" refers to a class of structurally related glycoproteins consisting of two pairs of polypeptide chains, one pair of light (L) low molecular weight chains and one pair of heavy (H) chains, all four potentially inter-connected by disulfide bonds. The structure of immunoglobulins has been well characterized. See for instance Fundamental Immunology Ch. 7 (Paul, W., ed., 2nd ed. Raven Press, N.Y. (1989)). Briefly, each heavy chain typically is comprised of a heavy chain variable region (abbreviated herein as VH) and a heavy chain constant region. The heavy chain constant region typically is comprised of three domains, CH1, CH2, and CH3. The heavy chains are inter-connected via disulfide bonds in the so-called "hinge region". Each light chain typically is comprised of a light chain variable region (abbreviated herein as VL) and a light chain constant region. The light chain constant region typically is comprised of one domain, CL. The VH and VL regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4 (see also Chothia and Lesk J. Mol. Biol. 196, 901 917 (1987)). Unless otherwise stated or contradicted by context, CDR sequences herein are identified according to IMGT rules using DomainGapAlign (Lefranc MP., Nucleic Acids Research 1999;27:209-212 and Ehrenmann F., Kaas Q. and Lefranc M.-P. Nucleic Acids Res., 38, D301-307 (2010); see also internet http address (www.imgt.org/).

Reference to amino acid positions in the Fc region/Fc domain in the present invention is according to the EU-numbering (Edelman et al., Proc Natl Acad Sci USA. 1969 May;63(1):78-85; Kabat et al., Sequences of proteins of immunological interest. 5th Edition - 1991 NIH Publication No. 91-3242).

The Fc-region of an immunoglobulin is defined as the fragment of an antibody which would be typically generated after digestion of an antibody with papain and which includes the two CH2-CH3 regions of an immunoglobulin and a connecting region, e.g. a hinge region. The constant domain of an antibody heavy chain defines the antibody isotype, e.g. IgG1, IgG2, IgG3, IgG4, IgA1, IgA2, IgM, IgD, or IgE. The Fc-region mediates the effector functions of antibodies with cell surface receptors called Fc receptors and proteins of the complement system.

The term "glycosylation", as used herein, refers to the post-translational modification that involves selective attachment of glycans to a polypeptide, e.g. an antibody, in order to stabilize the structure of the polypeptide or convey function to the polypeptide. Glycan structures vary from organism to organism, but generally comprise two or more GlcNAc moieties, a number of mannose moieties and optionally further carbohydrate moieties, such as galactose moieties, fucose moieties and sialic acid moieties. The most prevalent types of glycosylation, N-linked and O-linked glycosylation, occur in the endoplasmic reticulum and Golgi apparatus of eukaryotic cells, respectively. N-linked (or Asn-linked) glycosylation refers to the process of linking of carbohydrate structures to the side-chain nitrogen atom of asparagine (Asn) residues of a polypeptide, while O-linked glycosylation refers to the process of linking of carbohydrate structures to the side-chain oxygen atom of serine (Ser) or threonine (Thr) residues of a polypeptide. Immunoglobulins, such as IgG molecules, have a single conserved Asn-linked glycosylation site at amino acid position Asn297 in the CH2 domain. The term "deglycosylation" as used herein refers to the removal, e.g. enzymatic removal, of glycans from a polypeptide, e.g. from an antibody.

When used herein, the term "core GlcNAc moiety" or "core Asn-linked GlcNAc moiety" refers to an N-acetylglycosamine, also known as GlcNAc, moiety that is directly attached to an Asn acceptor residue in the polypeptide chain (and thus not via another moiety). In other words, it is the GlcNAc moiety that is most proximal to the polypeptide chain.

When used herein, the terms "fucose" or fucosylated" refer to fucose moieties that are part of the glycan structure of a glycoprotein. Fucose moieties may occur at various locations in a complex glycan structure. In the present context, the fucose moieties linked to the core GlcNAc moieties are of particular interest.

When used herein, the term "full removal", or variations thereof, in the context of removal of Asn-linked glycans from glycoproteins refers to removal of the entire glycan structure from an Asn acceptor site in the polypeptide chain, including the core GlcNAc moieties. The Asn acceptor residue is thereby converted to Asp.

The term "hinge region" as used herein is intended to refer to the hinge region of an immunoglobulin heavy chain. Thus, for example the hinge region of a human IgG1 antibody corresponds to amino acids 216-230 according to the EU numbering.

The term "CH2 region" or "CH2 domain" as used herein is intended to refer to the CH2 region of an immunoglobulin heavy chain. Thus, for example the CH2 region of a human IgG1 antibody corresponds to amino acids 231-340 according to the EU numbering. However, the CH2 region may also be any of the other subtypes as described herein.

The term "CH3 region" or "CH3 domain" as used herein is intended to refer to the CH3 region of an immunoglobulin heavy chain. Thus, for example the CH3 region of a human IgG1 antibody corresponds to amino acids 341-447 according to the EU numbering. However, the CH3 region may also be any of the other subtypes as described herein.

The term "isotype" as used herein, refers to the immunoglobulin class (for instance IgG1, IgG2, IgG3, IgG4, IgD, IgA1, IgGA2, IgE, or IgM or any allotypes thereof such as IgG1m(za) and IgG1m(f)) that is encoded by heavy chain constant region genes. Further, each heavy chain isotype can be combined with either a kappa (κ) or lambda (λ) light chain.

The term "antibody" (Ab) in the context of the present invention refers to an immunoglobulin molecule, a fragment of an immunoglobulin molecule, or a derivative of either thereof, which has the ability to specifically bind to an antigen. The variable regions of the heavy and light chains of the immunoglobulin molecule contain a binding domain that interacts with an antigen. As described above, the constant or "Fc" regions of the antibodies may mediate the binding of the immunoglobulin to host tissues or factors, including various cells of the immune system (such as effector cells) and components of the complement system such as C1q, the first component in the classical pathway of complement activation. An antibody may also be a multispecific antibody, such as a bispecific antibody or similar molecule. The term "bispecific antibody" refers to an antibody having specificities for at least two different, typically non-overlapping, epitopes. Such epitopes may be on the same or different targets. If the epitopes are on different targets, such targets may be on the same cell or different cells or cell types. As indicated above, unless otherwise stated or clearly contradicted by the context, the term antibody herein includes fragments of an antibody which retain the ability to specifically bind to the antigen. Such fragments may be provided by any known technique, such as enzymatic cleavage, peptide synthesis and recombinant expression techniques. It has been shown that the antigen-binding function of an antibody may be performed by fragments of a full-length antibody.

The term "full-length antibody" when used herein, refers to an antibody that contains all heavy and light chain constant and variable domains corresponding to those that are normally found in a wild-type antibody of that isotype. A "full length amino acid sequence" refers to the entire amino acid sequence of a protein as it occurs in the sample to be quantified, e.g. the entire amino acid sequence of the heavy chain of an antibody in the sample.

The terms "antigen-binding region", "antigen binding region", "binding region" or "antigen binding domain", as used herein, refer to a region of an antibody that is capable of binding to the antigen. This binding region is typically defined by the VH and VL domains of the antibody which may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). The antigen can be any molecule, such as a polypeptide, e.g. present on a cell, bacterium, or virion.

The term "target" or "target antigen" as used herein, refers to a molecule to which the antigen-binding region of the antibody binds. The target includes any antigen towards which the antibody is directed. The term "antigen" and "target" may in relation to an antibody be used interchangeably and constitute the same meaning and purpose with respect to any aspect or embodiment of the present invention.

A "variant form" is a glycoprotein molecule which comprises one or more modifications, e.g. amino acid substitutions, as compared to a "parent" glycoprotein. Exemplary parent antibody formats include, without limitation, a wild-type antibody, a full-length antibody or Fc-containing antibody fragment, a bispecific antibody, a human antibody, humanized antibody, chimeric antibody or any combination thereof. The modifications may comprise modifications of the glycans or of the amino acid sequence. In the context of the present invention, an amino acid substitution in a variant is indicated as:
Original amino acid - position - substituted amino acid;

The three-letter code, or one letter code, are used, including the codes Xaa and X to indicate amino acid residue. Accordingly, the notation "E345R" or "Glu345Arg" means that the variant comprises a substitution of glutamic acid with arginine in the variant amino acid position corresponding to the amino acid in position 345 in the parent antibody.

The term "recombinant host cell" (or simply "host cell"), as used herein, is intended to refer to a cell into which an expression vector has been introduced. It should be understood that such terms are intended to refer not only to the particular subject cell, but also to the progeny of such a cell. Because certain modifications may occur in succeeding generations due to either mutation or environmental influences, such progeny may not, in fact, be identical to the parent cell, but are still included within the scope of the term "host cell" as used herein. Recombinant host cells include, for example, CHO cells, HEK-293 cells, PER.C6, NS0 cells, and lymphocytic cells and other eukaryotic hosts such as plant cells and fungi.

The term "plasma half-life" as used herein indicates the time it takes to reduce the concentration of polypeptide in the blood plasma to one half of its initial concentration during elimination (after the distribution phase). For antibodies the distribution phase will typically be 1-3 days, during which there is about 50% decrease in blood plasma concentration due to redistribution between plasma and tissues.

The term "antibody-drug conjugate", as used herein refers to an antibody or Fc-containing polypeptide having specificity for at least one type of malignant cell, a drug, and a linker coupling the drug to e.g. the antibody. The linker is cleavable or non-cleavable in the presence of the malignant cell, wherein the antibody-drug conjugate kills the malignant cell.

### Further aspects and embodiments of the invention

As described above, in a first aspect, the invention relates to a method for the quantification of one or more glycoproteins in a sample, wherein each glycoprotein to be quantified comprises one or more Asn-linked glycans, said method comprising the steps of:
a. providing a sample comprising said one or more glycoproteins to be quantified,
b. adding an internal standard to said sample, wherein said internal standard comprises a variant form of each of said one or more glycoproteins to be quantified, wherein said variant form is a form containing only core Asn-linked GlcNAc moieties, each core Asn-linked GlcNAc moiety being an N-acetylglycosamine moiety that is directly attached to an Asn acceptor residue in the polypeptide chain of said variant, and wherein no fucose moieties are linked to said core GlcNAc moieties; and
c. quantifying said one or more glycoproteins by comparison with the internal standard.

In one embodiment, the method of the invention does not comprise a step of proteolytically digesting, or otherwise fragmenting, the polypeptide chain of the glycoproteins to be quantified.

In one embodiment, the method comprises, prior to step c., a treatment of the sample with an enzyme, wherein said enzyme is capable of fully removing Asn-linked glycans from said one or more glycoproteins, but is not capable of removing core Asn-linked GlcNAc moieties from said variant form, i.e. the enzyme is capable of fully removing Asn-linked glycans extending beyond the core GlcNAc moiety, but is not capable of removing Asn-linked glycans with no extension beyond the core GlcNAc moiety, such as Asn-linked glycans only consisting of the, optionally fucosylated, core GlcNAc moiety.

As shown in the Examples herein, PNGase F is an example of an enzyme which can fully remove Asn-linked glycans from glycoproteins, i.e. this enzyme removes the entire Asn-linked glycans from the glycoproteins, including the core GlcNAc moieties (thereby converting the Asn to Asp). However, it is also shown in the Examples herein that PNGase F does not remove core GlcNAc moieties from glycoprotein variants that only have core Asn-linked GlcNAc moieties. Thus, treatment of the sample with an enzyme having the properties of PNGase F results in full deglycosylation of the glycoproteins in the sample, but not in full deglycosylation of the internal standard which retains the core Asn-linked GlcNAc. The method of the invention exploits the resulting difference in mass for quantification of the protein in the sample relative to the internal standard. Figure 1 illustrates a (non-limiting) embodiment of the method of the invention.

In one embodiment, the treatment with the enzyme that is capable of fully removing Asn-linked glycans from said one or more glycoproteins, but is not capable of removing core Asn-linked GlcNAc moieties from the variant form, is performed prior to step b. In another embodiment, said enzyme treatment is performed after addition of the internal standard in step b. In one embodiment, said enzyme is Peptide:N-glycosidase F, otherwise known as PNGase F (EC 3.5.1.52) (Norris et al., Structure 2(11): p1049-59 (1994)).

In another embodiment, the method comprises, prior to step b., a step of treating the sample with an enzyme capable of fully removing Asn-linked glycans from said one or more glycoproteins, optionally followed by removal or deactivation of said enzyme prior to step b. For example, if, for the deglycosylation of the glycoprotein to be quantified, an enzyme is used which can fully remove Asn-linked glycans from the glycoprotein, but can also remove core Asn-linked GlcNAc from the variant form, then it may be beneficial or even necessary to remove or inactivate said enzyme before addition of the variant form in order to preserve the difference in mass between the variant form and the deglycosylated proteins to be quantified.

As described, the method of the invention uses variant forms of the glycoproteins to be quantified as internal standard. Preferably, for each glycoprotein to be quantified, the corresponding variant form in the internal standard is a homogeneous preparation, i.e. for each glycoprotein, all variant molecules are identical in mass.

The variant form used is a form containing only core Asn-linked GlcNAc moieties. In particular, each core Asn-linked GlcNAc moiety may consist of a single N-acetylglycosamine moiety that is directly attached to an Asn acceptor residue in the polypeptide chain of said variant form of each of said one or more glycoproteins. In a preferred embodiment, no fucose moieties are linked to said core GlcNAc moieties.

Said core GlcNAc moieties may be fucosylated, preferably 100% fucosylated, i.e. all molecules of the variant form used as internal standard are fucosylated.

The variant form used as internal standard may be more than 95% glycosylated, i.e. more than 95% of the Asn acceptor sites of the total number of acceptor sites of all variant molecules in the composition have a GlcNAc attached. Further, the variant form may be more than 98%, such as more than 99%, e.g. 100% glycosylated.

As described, the quantification of said one or more glycoproteins is performed by comparison with the internal standard. This overcomes the problem that an external calibration curve, when used exclusively, consisting of samples that are measured as separate measurements, is influenced by experimental conditions such as MS instrument conditions, spray conditions, sample recovery, carry-over, etc. Both the internal standard and sample analyte are analyzed simultaneously to compensate for any possible variation during the entire process of sample preparation and analysis. The added amount of the internal standard should be in an appropriate ratio to the analyte after consideration of the dynamic range. No calibration curve is required as long as the concentrations of internal standard and the sample analyte(s) fall in the linear dynamic range. However, the use of an external calibration curve in conjunction with internal standards is common practice and provides the best results as the use of external standards (ES) will not have the interferences of e.g. endogenous proteins and/or sample matrices and the internal standard simultaneously corrects for any bias in instrument stability during analysis. To obtain a calibration curve, MS peak intensity ratios (ES/IS) are calculated (Y-values) and plotted against known ES concentrations (X-values). Upon sample analysis (spiked with IS), MS peak intensity ratios (Y-values) can be calculated (Unknown/IS) and sample concentrations can be deduced from the calibration curve by interpolation.

In one embodiment, the quantification in step c. is performed using mass spectrometry. In a further embodiment, the quantification in step c. is performed using a combination of mass spectrometry and a non-mass-based separation technique. Such a combination of different techniques can in particular be useful if the sample has a complex composition and/or more than one glycoprotein is to be quantified. The non-mass-based separation technique may allow separation of two or more proteins having a highly similar mass, thus enabling separate quantification. For example, the sample may, after addition of the internal standard, comprise two or more glycoproteins having less than 75 Da, such as less than 60 Da, e.g. less than 50 Da, difference in mass. In one embodiment, the quantification in step c. is performed using liquid chromatography-mass spectrometry (LC-MS), such as reverse-phase-based LC-MS.

The glycoproteins to be quantified may be any glycoprotein comprising one or more Asn-linked glycans, including natural glycoproteins, recombinant glycoproteins, therapeutic glycoproteins, diagnostic glycoproteins, enzymes, etc.The glycoproteins to be quantified may contain only Asn-X-Thr glycosylation sites and no Asn-X-Ser sites.The glycoproteins to be quantified may be more than 95% glycosylated, i.e. more than 95% of the Asn acceptor sites of the total number of acceptor sites of all molecules in the composition have a glycan attached. Further, the glycoproteins to be quantified may be more than 98%, such as more than 99%, e.g. 100% glycosylated.

In some embodiments, two or more, such as two, three, four, five or more different glycoproteins are being quantified, preferably simultaneously. In particular, the sample provided may comprise 2-10 different antibodies, such as 2-9, 2-8, 2-7, 2-6, 2-5, 2-4, 2-3, 3-10, 3-9, 3-8, 3-7, 3-6, 3-5, 3-4, 4-10, 4-9, 4-8, 4-7, 4-6, 4-5, 5-10, 5-9, 5-8, 5-7, 5-6, 6-10, 6-9, 6-7, 7-10, 7-9, 7-8, 8-10, 8-9, or 9-10 different antibodies. In particular, the sample may comprise two or more antibodies to be quantified, wherein the least abundant of said two or more different antibodies is present in an amount which is at least 1% (w/w), 2% (w/w), 3% (w/w), 4% (w/w), 5%(w/w), 6% (w/w), 7% (w/w), 8%(w/w), 9% (w/w) or 10% (w/w) of the amount of the most abundant of the said two or more different antibodies. In particular, the two or more antibodies may be present in such amounts that the ratio (w/w) between the amounts of any two antibodies is between 1:5 and 5:1, such as between 1:4 and 5:1, 1:3 and 5:1, 1:2 and 5:1, 1:1 and 5:1, 2:1 and 5:1, 3:1 and 5: 1, 3:4 and 5:1, 1:5 and 4:1, 1:5 and 3:1, 1:5 and 2:1, 1:5 and 1:1, 1:5 and 1:2, 1:5 and 1:3, 1:5 and 1:4, 1:4 and 4:1, 1:4 and 3:1, 1:4 and 2:1, 1:4 and 1:1, 1:4 and 1:2, 1:4 and 1:3, 1:3 and 4:1, 1:3 and 3:1, 1:3 and 2:1, 1:3 and 1:1, 1:3 and 1:2, 1:2 and 4:1, 1:2 and 3:1, 1:2 and 2:1, 1:2 and 1:1, 1:1 and 4:1, 1:1 and 3:1, or such as between 1:1 and 2:1.

Natural glycoproteins to be quantified may be of any origin, including e.g. animal origin, such as mammalian origin, or other eukaryotic origin, such as fungal or yeast origin. Similarly, recombinantly produced glycoproteins may be produced in any kind of host cell that is capable of Asn-linked glycosylation, including animal host cells, such as mammalian host cells, such as CHO cells, or human cells, such as HEK cells, or fungal or yeast cells.

In one embodiment, the one or more glycoproteins to be quantified are antibodies. In a further embodiment, the method comprises quantification of two or more, such as two, three, four, five or more different antibodies. In an even further embodiment, said two or more different antibodies are IgG antibodies, such as full-length IgG antibodies. "different", in this context, can be a difference in amino-acid sequence and/or a difference in any post-translational modification other than Asn-linked glycosylation, e.g. conjugation, terminal clipping or other amino acid residue modifications. The difference may be a difference in a post-translational modification other than Asn-linked glycosylation.The method may comprise simultaneous quantification of an antibody-drug conjugate and a non-conjugated antibody in the same sample.

The samples containing one or more glycoproteins to be quantified may be samples obtained from one of the steps of a method described in the art to generate bispecific antibodies in vitro via half-molecule exchange (see e.g. Labrijn et al. (2013) PNAS 110:5145 and WO2011131746). In some of these methods, homodimeric parental antibodies are mixed and subjected to controlled reducing conditions in vitro that separate the antibodies into half-molecules and allow reassembly and reoxidation to form bispecific antibodies. The quantification method of the present invention can be used to monitor progress and/or assay purity of bispecific antibody formation, by simultaneous quantification of the homodimeric parental antibodies and the resulting bispecific antibodies.

The samples containing one or more glycoproteins to be quantified may be samples obtained from one of the steps of the method described in WO2019243626 (Genmab).

WO2019243626 describes a method for producing an output mixture of two or more different antibodies having a difference in their amino acid sequences, which difference enables separation of the antibodies by chromatography, wherein
- the two or more different antibodies are present in said output mixture at, or essentially at, a desired or predetermined concentration ratio; and
- the method comprises the steps of:
   a. providing an input mixture wherein the two or more different antibodies are not present at, or essentially at, the desired or predetermined concentration ratio;
   b. separating the two or more antibodies by chromatography;
   c. recovering the two or more antibodies in the amounts required to provide the output mixture.

The term "output mixture" herein is intended to refer to an antibody mixture, wherein the two or more different antibodies are present at a desired or predetermined concentration ratio. The term "input mixture" is intended to refer to an antibody mixture, wherein at least two of the two or more different antibodies referred to in the context of the "output mixture" are present at a concentration ratio which is not the desired or predetermined concentration ratio and/or is not within the tolerated deviation from the desired or predetermined concentration ratio.

Thus, the method of the present invention may e.g. be used to quantify antibodies in the input mixture or the output mixture.

The sample provided in the method of the invention may be any kind of sample comprising glycoproteins. In one embodiment, the sample is a cell culture sample, i.e. a sample taken from, or derived from, a culture of cells, e.g. recombinant host cells, that produce the glycoprotein(s) to be quantified. The method of the invention may thus e.g. be used to monitor production of glycoproteins, including, if more than one glycoprotein is produced in the cell culture, the monitoring of the quantity and/or ratio of the glycoproteins, e.g. antibodies produced.

Accordingly, in a further aspect, the invention relates to a process for monitoring production of glycoproteins in a cell culture, said process comprising culturing host cells producing said glycoproteins and performing the method according to the invention as described herein. Depending on the outcome of the quantification, the cell culture growth conditions may be adjusted to modulate glycoprotein production or, if more than one glycoprotein is produced, the ratio of the glycoproteins.

The method of the invention may also be used for quality control (GC) of glycoprotein preparation or batches after purification and/or polishing. Thus, in one embodiment, the sample comprises purified glycoproteins, such as purified recombinantly produced glycoproteins.

Accordingly, in a further aspect, the invention relates to a process for the quality control of purified recombinantly produced glycoproteins, said process comprising performing the method according to the invention as described herein.

The method according to the invention may be carried out in an automated manner, i.e. without human intervention in one or all steps of the method. In one embodiment, steps b. and c. of the method are performed in an automated manner. In a further embodiment, steps a., b. and c. are performed in an automated manner. The method may provide quantification results in a sufficiently short time-frame, e.g. 4 hours or less, to allow adjustment of an ongoing cell culture process.

In another embodiment, the sample is, or is derived from, a bodily fluid sample, such as a blood sample, plasma sample or serum sample. The method of the invention may thus be used to monitor the fate, e.g. the plasma half-life, of the glycoprotein(s) in a subject, such as a human subject.

The method of the invention is suitable for quantifying glycoproteins across a wide range of concentrations. The concentration of each of the glycoproteins to be quantified in the sample may be above 0.001 g/L, such as above 0.005 g/L, e.g. above 0.01 g/L. Alternatively, the concentration of each of the glycoproteins to be quantified in the sample is below 1 g/L, such as below 0.25 g/L, e.g. below 0.1 g/L.

As described, in a further aspect, the invention relates to a method for the quantification of one or more glycoproteins in a sample, wherein each glycoprotein to be quantified comprises one or more Asn-linked glycans, said method comprising the steps of:
a. preparing an internal standard by treating said one or more glycoproteins with one or more enzymes to obtain a variant form of each of said one or more glycoproteins, wherein said variant form is a form containing only core Asn-linked GlcNAc moieties, each core Asn-linked GlcNAc moiety being an N-acetylglycosamine moiety that is directly attached to an Asn acceptor residue in the polypeptide chain of said variant, and wherein no fucose moieties are linked to said core GlcNAc moieties,
b. providing a sample comprising said one or more glycoproteins to be quantified,
c. adding said internal standard to said sample, and
d. quantifying said one or more glycoproteins by comparison with the internal standard.

The method may *mutatis mutandis* comprise one or more of the further features described above for the method of the first aspect of the invention.

In one embodiment, the step of preparing the internal standard comprises treatment with an endo-β-N-acetylglucosaminidase (ENGase EC 3.2.1.96) (Fairbanks (2017) Chem Soc Rev46:5128), such as EndoS2 or EndoS.

In one embodiment, said treatment with one or more enzymes in step a. comprises treatment with an endo-β-N-acetylglucosaminidase and a fucosidase, capable of hydrolyzing alpha-1,6-linkage from an Asn-linked fucose-alpha-1,6-GlcNAc moiety, e.g. fucosidase 29A, also known as LcFuc29A (GH29), E.C. number 3.2.1.51from *Lactobacillus casei* or from *Bacteroides fragilis* (Tsai et al. 2017 ACS Chem. Biol 12:63). In a further embodiment, the step of preparing the internal standard comprises treatment with EndoS2 or EndoS (preferably EndoS2) followed by a treatment with a fucosidase.

At least one of the one or more glycoproteins to be quantified in the above described methods of the invention may contain fucose on core Asn-linked GlcNAc moieties, i.e. some of all of the core Asn-linked GlcNAc moieties.

The internal standard used in the methods of the invention may be a standard, which is not isotope labelled.

Both the glycoprotein to be quantified and the variant form thereof used in the internal standard may be used in their full-length forms, i.e. having a full-length amino acid chain, or almost full-length forms, e.g. comprising 50% or more, such as 75% or more, e.g. 90% or more, such as 95% or more of their full-length amino acid sequence, e.g. comprising 50% or more, such as 75% or more, e.g. 90% or more, such as 95% or more of a heavy-chain antibody sequence.
In a further aspect, the invention relates to the use of a composition comprising a known quantity of variant form of a glycoprotein, wherein said variant form is a form containing only core Asn-linked GlcNAc moieties, each core Asn-linked GlcNAc moiety being an N-acetylglycosamine moiety that is directly attached to an Asn acceptor residue in the polypeptide chain of said variant, and wherein no fucose moieties are linked to said core GlcNAc moieties as an internal standard for quantification of said glycoprotein in a sample.

### Sequences

| **SEQ ID NO** | **Name** | **Sequence** |
|---|---|---|
| **1** | constant region human HC IgG1m(f) | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVT CVVVDVSHEDPEVKFNWYVDGVEVHNAKTKPREEQYNSTYRVVSVL TVLHQDWLNGKEYKCKVSNKALPAPIEKTISKAKGQPREPQVYTLPPS REEMTKNQVSLTCLVKGFYPSDIAVEWESNGQPENNYKTTPPVLDSD GSFFLYSKLTVDKSRWQQGNVFSCSVMHEALHNHYTQKSLSLSPGK |
| **2** | constant region human HC IgG1m(f)-E345K | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVT CVVVDVSHEDPEVKFNWYVDGVEVHNAKTKPREEQYNSTYRVVSVL TVLHQDWLNGKEYKCKVSNKALPAPIEKTISKAKGQPRKPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG K |
| **3** | Constant region human kappa LC | |
| **4** | VH CD19-21D4 | EVQLVQSGAEVKKPGESLKISCKGSGYSFSSSWIGWVRQMPGKGL EWMGIIYPDDSDTRYSPSFQGQVTISADKSIRTAYLQWSSLKASDT AMYYCARHVTMIWGVIIDFWGQGTLVTVSS |
| **5** | VH CD19-21D4 CDR1 | GYSFSSSW |
| **6** | VH CD19-21D4 CDR2 | IYPDDSDT |
| **7** | VH CD19-21D4 CDR3 | ARHVTMIWGVIIDF |
| **8** | VL CD19-21D4 | AIQLTQSPSSLSASVGDRVTITCRASQGISSALAWYQQKPGKAPKLL IYDASSLESGVPSRFSGSGSGTDFTLTISSLQPEDFATYYCQQFNSY PYTFGQGTKLEIK |
| **9** | VL 1 CD19-21D4 CDR1 | QGISSA |
| | VL CD19-21D4 CDR2 | DAS |
| **10** | VL CD19-21D4 CDR3 | QQFNSYPYT |
| **11** | VH CD22-huRFB4 CDR1 | GFAFSIYD |
| **12** | VH CD22-huRFB4 CDR2 | ISSGGGTT |
| **13** | VH CD22-huRFB4 CDR3 | ARHSGYGSSYGVLFAY |
| **14** | VL 1 CD22-huRFB4 CDR1 | QDISNY |
| | VL CD22-huRFB4 CDR2 | YTS |
| **15** | VL CD22-huRFB4 CDR3 | QQGNTLPWT |
| **16** | VH 7D8 | EVQLVESGGGLVQPDRSLRLSCAASGFTFHDYAMHWVRQAPGKGL EWVSTISWNSGTIGYADSVKGRFTISRDNAKNSLYLQMNSLRAEDT ALYYCAKDIQYGNYYYGMDVWGQGTTVTVSS |
| **17** | VH 7D8 CDR1 | GFTFHDYA |
| **18** | VH 7D8 CDR2 | ISWNSGTI |
| **19** | VH 7D8 CDR3 | AKDIQYGNYYYGMDV |
| **20** | VL 7D8 | EIVLTQSPATLSLSPGERATLSCRASQSVSSYLAWYQQKPGQAPRLLI YDASNRATGIPARFSGSGSGTDFTLTISSLEPEDFAVYYCQQRSNW PITFGQGTRLEIK QSVSSY |
| **21** | VL 1 7D8 CDR1 | QSVSSY |
| | VL 7D8 CDR2 | DAS |
| **22** | VL 7D8 CDR3 | QQRSNWPIT |
| **23** | VH CD37-37-3 | QVQVKESGPGLVAPSQSLSITCTVSGFSLTTSGVSWVRQPPGKGLE WLGVIWGDGSTNYHSALKSRLSIKKDHSKSQVFLKLNSLQTDDTAT YYCAKGGYSLAHWGQGTLVTVSA |
| **24** | VH CD37-37-3 CDR1 | GFSLTTSG |
| **25** | VH CD37-37-3 CDR2 | IWGDGST |
| **26** | VH CD37-37-3 CDR3 | AKGGYSLAH |
| **27** | VL CD37-37-3 | DIQMTQSPASLSVSVGETVTITCRASENIRSNLAWYQQKQGKSPQL LVNVATNLADGVPSRFSGSGSGTQYSLKINSLQSEDFGTYYCQHYW GTTWTFGGGTKLEIK |
| **28** | VL 1 CD37-37-3 CDR1 | ENIRSN |
| | VL CD37-37-3 CDR2 | VAT |
| **29** | VL CD37-37-3 CDR3 | QHYWGTTWT |
| **30** | VH CD52-Campath | QVQLQESGPGLVRPSQTLSLTCTVSGFTFTDFYMNWVRQPPGRGLE WIGFIRDKAKGYTTEYNPSVKGRVTMLVDTSKNQFSLRLSSVTAAD TAVYYCAREGHTAAPFDYWGQGSLVTVSS |
| **31** | VH CD52-Campath CDR1 | GFTFTDFY |
| **32** | VH CD52-Campath CDR2 | IRDKAKGYTT |
| **33** | VH CD52-Campath CDR3 | AREGHTAAPFDY |
| **34** | VL CD52-Campath | DIQMTQSPSSLSASVGDRVTITCKASQNIDKYLNWYQQKPGKAPKL LIYNTNNLQTGVPSRFSGSGSGTDFTFTISSLQPEDIATYYCLQHISR PRTFGQGTKVEIK |
| **35** | VL 1 CD52-Campath CDR1 | QNIDKY |
| | VL CD52-Campath CDR2 | NTN |
| **36** | VL CD52-Campath CDR3 | LQHISRPRT |

The present invention is further illustrated by the following examples which should not be construed as further limiting.

### EXAMPLES

### Example 1: Expression and antibody production of human IgG1-CD19-21D4, human IgG1-CD22-huRFB4, human IgG1-7D8, human IgG1-CD37-37-3 and human IgG1-CD52-Campath and Variants

For antibody expression of isolated immunoglobulin proteins, variable heavy (VH) chain and variable light (VL) chain sequences were prepared by gene synthesis (GeneArt Gene Synthesis; ThermoFisher Scientific, Germany) and cloned in pcDNA3.3 expression vectors (ThermoFisher Scientific, US) containing IgG1m(f) allotype heavy chain (HC; SEQ ID NO: 1) and light chain (LC; SEQ ID NO: 3) constant regions. The heavy chain constant region amino acid sequences as used are identified in the sequence reference table below.

Desired mutations were introduced either by gene synthesis or site-directed mutagenesis. Antibodies mentioned in this application have VH and VL sequences derived from previously described IgG1-CD19-21D4 (WO2007002223; VH: SEQ ID NO 4; VL: SEQ ID NO 8), IgG1-CD22-huRFB4 (humanized variant of murine antibody published in Weber et al., J Immunol Res 2015, 561814 (2015)), IgG1-7D8 (WO2004/035607; VH: SEQ ID NO 16; VL: SEQ ID NO 20), IgG1-CD37-37-3 (WO2011/112978; VH: SEQ ID NO 23; VL: SEQ ID NO 27) and IgG1-CD52-Campath (Crowe et al., Immunology 87(1):105-110 (1992); VH: SEQ ID NO 30; VL: SEQ ID NO 34). The sequences are also provided herein. The IgG1-CD19-21D4 and IgG1-CD52-Campath antibodies used in the Examples harbored a mutation at amino acid position E345, E345K (SEQ ID NO: 2), which enhances Fc:Fc interactions between individual antibodies. As these antibody variants are monomeric in solution, the presence of these mutations does not affect the method described in the current application.

The sequences were either subcloned from the pcDNA 3.3 expression vectors into the in-house developed expression vector pGENpr6DGV (IgG1-CD19-21D4-E345K, IgG1-CD22-huRFB4, IgG1-CD37-37-3 and IgG1-CD52-Campath-E345K) or cloned into vector pCon7D8-DGV (for IgG1-7D8), expressing both ORF's from the same vector. These expression vectors contained both antibody open reading frames regulated by an upstream CMV promoter and downstream TK poly-A transcription termination signal, and a glutamine synthetase selection marker expressed under the control of an SV40 promoter fragment and a SV40 poly-A transcription termination signal. Vectors were transferred into cells of a CHO-K1 cell line (ECACC cat. nr. 85051005), adapted to suspension growth on chemically defined media, or to the cells of a CHO-K1SV cell line (ECACC cat. nr. 85051005), adapted to suspension growth on chemically defined media at 1 µg/1.0E+06 cells, by nucleofection (Lonza Nucleofector 2b) using Amaxa Solution V kit essentially according to the instructions of the manufacturer using program T020 (Lonza). Cells containing expression vectors were grown in 96-well plates in CD-CHO medium (Life Technologies/Thermo Scientific) containing GS EM supplement (Sigma) under MSX selection (Sigma) for 4 weeks, after which a panel of parental cultures displaying growth and IgG expression was expanded to larger volumes. Top producing clones were tested for IgG expression in an Ambr15 platform (TAP Biosystems), after which the best producing parentals were selected for inoculation of 500mL up to 3L bioreactors to supply IgG material. Cell cultures were harvested after 14 days and IgG containing supernatants were collected by filtration.

Antibodies were purified by protein A affinity chromatography. The cell culture supernatants were filtered over a 0.2 µM dead-end filter, followed by loading on a MabSelect SuRe column of the appropriate size (GE Healthcare) and elution of the antibodies with 0.1 M citric acid-NaOH, pH 3. The eluate was immediately neutralized with 2 M Tris-HCl, pH 9 and dialyzed overnight to 12.6 mM sodium phosphate, 140 mM NaCl, pH 7.4 (GE Healthcare). After dialysis, samples were sterile filtered over a 0.2 µM dead-end filter. Concentration of the purified IgGs was determined by absorbance at 280 nm. Purified proteins were analyzed by CE-SDS, HP-SEC and mass spectrometry.

### Example 2: Enzymatic deglycosylation of IgG antibodies by EndoS2 reveals sample heterogeneity caused by the presence of afucosylated species, while PNGase F fully deglycosylates IgG antibodies

For absolute quantification of IgG antibody clones within a complex protein mixture, mass spectrometry in combination with internal IgG antibody standards can be used. These internal standards should have similar biophysical characteristics to those of the sample analytes (e.g. similar ionization efficiencies and physicochemical properties), yet should be distinguishable from the sample analyte. The Asn-linked glycosylation site Asn297 harbors structurally diverse Asn-linked glycan branches, all with two N-Acetylglucosamine (GlcNAc) groups at the base. The endoglycosidase EndoS2 hydrolyzes the linkage between these two GlcNAc groups, with only the Asn-linked core GlcNAc moiety that is most proximal to the polypeptide chain remaining. Here, it was tested whether enzymatic treatment using EndoS2 allows generation of internal standard antibodies in which a small, homogeneous mass tag is retained.

To hydrolyze the linkage between the two GlcNAc residues at the base of the N-glycan core of the Fc-glycan, 50 µL of 200 µg/mL antibody IgG1-CD19-21D4-E345K was treated with 1 µL of EndoS2 (40 U/µL; Genovis, cat. no. A0-GL1-020), an endoglycosidase from a serotype M49 group A *Streptococcus* strain, at 37°C for 1 hour. Alternatively, for full deglycosylation, 50 µL of 200 µg/mL IgG1-CD19-21D4-E345K was treated overnight with 1 µL of the peptide-N-glycosidase F (PNGase F; 2.5U/mL; PROzyme, cat. no. GKE-5006D) at 37°C.

Samples were analyzed by liquid chromatography-mass spectrometry (LC-MS), performed on a Dionex Ultimate 3000 UHPLC system (Thermo Scientific) coupled online to an Orbitrap Q-Exactive Plus mass spectrometer (Thermo Scientific). Samples were injected (5 µL) onto a BioResolve reversed-phase monoclonal antibody column (1.0 mm × 5 cm, 2.7 µm particle size with 450 Å pore size; Waters, cat. no. 186009015). The mobile phase A contained 0.1% formic acid (v/v) in water (BioSolve, cat. no. 0023244101BS), and mobile phase B contained 0.1% (v/v) formic acid in acetonitrile (BioSolve, cat. no. 0001934101BS). The proteins were eluted at 200 µL/min using the following linear gradient: 25% B at 0 min, 30% B at 2 min, 40% B at 12 min, 80% B at 13 min, 80% B at 18 min, 25% B at 19 min, 25% B at 25 min. Full MS spectra were acquired at a resolution setting of 17,500 with an automatic gain control (AGC) target value of 5 × 10⁶, and a maximum injection time of 200 ms. Each of the spectra consisted of 10 microscans.

The raw data was analyzed using the Genedata Expressionist Refiner MS (Version 13.0) software. The retention time (RT) range was restricted from 1 to 22 minutes, and the m/z range was restricted from 1400 to 4000 Da. The spectra were deconvoluted using the Harmonic Suppression Deconvolution of the intact protein activity (mode: fully automated, eagerness: standard; multiple retention times per mass: true; step: 1.0 Da) with curvature-based peak detection (smoothing: 7 scans; center computation: intensity-weighed (0% threshold); boundary determination: inflection points) and the following advanced settings: minimal peak intensity: 1%; minimal search mass: 5.0 kDa; maximal search mass: 500.0 kDa; relative mass window: 2%; maximal mass window: 1.0 kDa; mass deconvolution: relative mass window 10%, maximal mass window 5.0 kDa). The deconvoluted spectra were then averaged using the Arithmetic Mean method. Peaks of the deconvoluted spectra were detected using the spectrum peak detection activity (smoothing: 3 points; peak detection: ascent-based; center computation: local maximum; boundary determination: inflection points). Peaks with intensities lower than 1% of the maximal intensity were excluded.

Enzymatic treatment of IgG1-CD19-21D4-E345K with EndoS2 resulted in efficient removal of the glycans up to the Asn-linked GlcNAc group, leaving only the innermost GlcNAc residue and a single attached core fucose (IgG-(GlcNAc-Fuc)₂; Figure 2). However, the presence of antibody species without core fucosylation (i.e., without the core fucose group attached to the innermost GlcNAc residue via an α-1,6-linkage) in antibody preparations results in sample heterogeneity: in addition to IgG-(GlcNAc-Fuc)₂ antibodies, the EndoS2-treated sample also contains a fraction of antibodies containing one heavy chain with the innermost GicNAc residue linked to the core fucose and one heavy chain with the innermost GicNAc residue without the core fucose (-Fuc, or IgG-(GlcNAc-Fuc)), and a fraction of antibodies containing two heavy chains with the innermost GicNAc residues without the core fucose groups (-Fuc₂, or IgG-(GlcNAc)). In contrast, enzymatic treatment of antibody IgG1-CD19-21D4-E345K with PNGase F (Figure 2B) resulted in a homogeneous population of fully deglycosylated IgG1-CD19-21D4-E345K.

### Example 3: Enzymatic defucosylation of EndoS2-treated IgG antibodies with the Lactobacillus casei α-L-fucosidase removes the fucose group from the core GicNAc group

As shown in Example 2, enzymatic deglycosylation of IgG antibodies with EndoS2 results in a heterogeneous population of IgG antibodies carrying glycan groups consisting of a GicNAc core group with or without a fucose group attached. As a result, antibodies may contain two heavy chains with the innermost GlcNAc residue linked to a core fucose group (IgG-(GlcNAc-Fuc)₂), or alternatively, one or both of the heavy chains may harbor a core GlcNAc group without a fucose group attached (IgG-(GlcNAc-Fuc) or IgG-(GlcNAc), respectively).

Here, it was tested whether enzymatic defucosylation could be used to remove this heterogeneity by removing the residual fucose groups on IgG-(GlcNAc-Fuc)₂ and IgG-(GlcNAc-Fuc), while leaving the GlcNAc group untouched.

50 µL of 200 µg/mL IgG1-CD19-21D4-E345K was treated for 6 hours at 37°C with 1 µL of the endoglycosidase EndoS2 (40 U/µL; Genovis, cat. no. A0-GL1-020) to hydrolyze the linkage between the two GlcNAc residues at the base of the N-glycan core of the Fc-glycan, and with 1 µL of the *Lactobacillus casei* fucosidase (Fucosidase 29A; 0.5 mg/ml; NZYtech, cat. no. CZ0566) to hydrolyze the α-1,6 linkage of the core fucose group to the innermost GlcNAc residue. Alternatively, 50 µL of 200 µg/mL IgG1-CD19-21D4-E345K was treated for 6 hours at 37°C with 1 µL EndoS2. LC-MS was performed as described in Example 2.

Enzymatic treatment of EndoS2-treated IgG antibodies with o-L-fucosidase from *L. casei* efficiently removed the residual fucose groups from IgG-(GlcNAc-Fuc)₂ and IgG-(GlcNAc-Fuc), resulting in IgG antibodies with a homogeneous GlcNAc tag (Figure 3B).

### Example 4: PNGase F from PNGase F-treated samples does not remove the residual GlcNAc moiety from IgG antibodies treated with EndoS2 and α-L-fucosidase

As shown in Example 3, enzymatic deglycosylation of IgG antibodies with EndoS2 and o-L-fucosidase leaves a small, homogeneous GicNAc moiety on both heavy chains. When used as internal standards, these IgG-(GlcNAc)₂ antibodies can be spiked into a complex mixture of antibodies that have been fully deglycosylated with the endoglycosidase PNGase F. We tested if PNGase F could remove the residual GicNAc moieties from IgG-(GlcNAc)₂ antibodies, to determine if depletion or inactivation of PNGase F from antibody samples would be required before the internal IgG-(GlcNAc)₂ standards can be spiked in.

50 µL of 400 µg/mL IgG1-CD19-21D4-E345K was treated for 6 hours at 37°C with 2 µL of the endoglycosidase EndoS2 (40 U/µL; Genovis, cat. no. A0-GL1-020) to hydrolyze the linkage between the two GlcNAc residues at the base of the N-glycan core of the Fc-glycan, and with 2 µL of the *Lactobacillus casei* fucosidase (Fucosidase 29A; 0.5 mg/ml; NZYtech, cat. no. CZ0566) to hydrolyze the α-1,6 linkage of the core fucose group to the innermost GlcNAc residue. In parallel, 50 µL of 400 µg/mL IgG1-CD19-21D4-E345K was fully deglycosylated overnight at 37°C with 2 µL of PNGase F (2.5 U/mL) as described in Example 2. After the treatment, 25 µL of each sample was mixed 1:1. LC-MS was performed as described in Example 2.

Alternatively, 50 µL of 600 µg/mL IgG1-CD19-21D4-E345K was treated with 3 µL EndoS2 and 3 µL o-L-fucosidase for 6 hours at 37°C. In parallel, 50 µL of 600 µg/mL IgG1-CD19-21D4-E345K was fully deglycosylated overnight at 37°C with 3 µL PNGase F (2.5 U/mL). 25 µL of each sample and 25 µL of 600 µg/mL IgG1-b12 were mixed 1:1:1 and incubated for 3 hours at 37°C. LC-MS was performed as described in Example 2.

The addition of PNGase F-treated IgG1-CD19-21D4-E345K to EndoS2- and o-L-fucosidase-treated IgG antibodies (IgG1-CD19-21D4-E345K-(GlcNAc)₂) did not result in the removal of the residual GlcNAc moiety on the EndoS2- and α-L-fucosidase-treated IgG antibodies (Figure 4A), showing that inactivation or removal of PNGase F from the sample analyte is not required.

The addition of PNGase F-treated IgG1-CD19-21D4-E345K to the EndoS2- and o-L-fucosidase-treated internal standard IgG1-CD19-21D4-E345K and the untreated IgG1-b12 did not result in full deglycosylation of the internal standard or untreated IgG1-b12. Instead, IgG1-b12 appears as the IgG1-b12-(GlcNAc)2 and IgG1-b12-(GlcNAc)-(GlcNAc-Fuc) variants, suggesting that the enzymatic activities of EndoS2 and α-L-fucosidase from the internal standard dominate the enzymatic activity of PNGase F from the sample analyte in this mixture.

### Example 5: Analysis of antibody mixtures is facilitated by multi-dimensional separation methods

As described in Examples 2-4, highly homogeneous variant antibody internal standards can be generated through subsequent deglycosylation of antibody samples by an EndoS2 endoglycosidase and *Lactobacillus casei* fucosidase (29A). The use of this method results in intact whole proteins having only a residual Asn-linked core GlcNAc moiety on both heavy chains. These antibody variants can be used as internal standards for the quantitation of individual antibodies in antibody mixtures. Mass spectrometry-based separation and detection of samples containing antibodies, however, is most optimal when all analyzed antibody samples and their designated internal standards do not have overlapping mass profiles i.e. (near) isobaric masses. Here, we describe how an LC-based separation method can be added to the procedure to enable time-resolved deconvolution of samples containing antibody mixtures with overlapping mass profiles (i.e. (near) isobaric masses).

An antibody mixture was generated by mixing equal concentrations of the human antibodies IgG1-CD19-21D4-E345K, IgG1-CD22-huRFB4, IgG1-7D8, IgG1-CD37-37-3 and IgG1-CD52-Campath-E345K to a final concentration of 8 mg/mL total. For the generation of the internal standards, 40 µL of EndoS2 (40 U/µL; Genovis, cat. no. A0-GL1-020), as well as 21.3 µL of *Lactobacillus casei* fucosidase (Fucosidase 29A; 0.5 mg/ml; NZYtech, cat. no. CZ0566) were added to 200 µL of the antibody mixture, as well as 378.6 µL MilliQ (final concentration 2.5 mg/mL), and incubated overnight at 37°C. The sample antibody mixture was fully deglycosylated by adding 160 µL of PNGase F (2.5U/mL; PROzyme, cat. no. GKE-5006D) to 200 µL of the antibody mixture, as well as 440 µL MilliQ (final concentration 2 mg/mL) and incubating the sample overnight at 37°C. After diluting the antibody sample to a concentration of 120 µg/mL, the internal standards were added to a final concentration of 75 µg/mL. LC-MS was performed essentially as described in Example 2.

The raw data was analyzed using the Genedata Expressionist Refiner MS (Version 13.0) software. The retention time (RT) range was restricted from 1 to 16 minutes, followed by alignment of the chromatograms using a pairwise alignment-based tree alignment scheme with a gap penalty of 1 and a maximal RT shift of 50 scans. Chemical noise was subtracted using the chromatogram chemical noise subtraction activity (RT window: 50; quantile: 50%, method: clipping, threshold: 0.0, RT structure removal: 6 scans). The ion map was deconvoluted using Time-resolved Deconvolution of the intact protein activity (minimal mass: 140 kDa; maximal mass: 150 kDa; method: Harmonic Suppression Deconvolution; 1.0 Da steps; 7 scans, curvature-based, intensity-weighed (0% threshold) peak detection with inflection points to determine the boundaries; minimal peak intensity: 1%; minimal search window: 5.0 kDa; maximal search window: 500.0 kDa; relative mass window: 2%; maximal mass window: 1.0 kDa; and mass deconvolution with a relative mass window of 10% and a maximal mass window of 5.0 kDa). Peaks of the deconvoluted ion map were detected using the peak detection activity (summation window: 10 scans; minimum peak size: 10 scans; maximum merge distance: 25 points; merge strategy: centers; smoothing: 3 points; peak detection: ascent-based; isolation filter: true, 3 points; center computation: local maximum; boundary determination: inflection points). Prior to the detection, the peaks were identified by the Protein mapping activity using the known sequences (mass tolerance of 10 Da with consolidate matches of 10 Da; fixed modification of N-terminal glutamine to pyroglutamate and of C-terminal loss of a lysine; glycosylation settings: deglycosylated, only Asn-linked sites, use of consensus sequences and filtering for core structures; fully connected disulfide bridges; complex connectivity for each IgG; and the following mass shifts for conjugates: GlcNAc(Fuc)₂: 698 Δ; GlcNAc(Fuc): 552 Δ; GlcNAc: 406 Δ , with a maximal number of 1 conjugate).

Figure 5A shows the mass spectrum of the antibody mixture composed of human IgG1-CD19-21D4-E345K, IgG1-CD22-huRFB4, IgG1-7D8, IgG1-CD37-37-3 and IgG1-CD52-Campath-E345K (labeled as species A - E), and their corresponding internal standards (species A' - E', all indicated with an apostrophe). Antibody sample B (IgG1-CD22-RFB4) and D (IgG1-CD37-37-3) have a near-identical mass and therefore their mass peaks overlap. Similarly, their corresponding internal standards B' and D' have overlapping mass peaks. Mass spectra of the other antibody samples and their corresponding internal standards do not show overlap. LC separation results in 4 major elution peaks (Figure 5B), making it difficult to identify the individual antibody samples and internal standards. However, by applying Time-Resolved Deconvolution (TRD) in which the mass (x-axis) and elution time (y-axis) were plotted, a two-dimensional view could be generated allowing the identification of the individual antibody samples and internal standards (Figure 5C). Despite the overlapping mass peaks of antibody B and D and accompanied internal standards B' and D', the difference in elution time between these samples enabled peak separation after TRD. In the third dimension peak intensities are preserved in such a way that peak volumes can be deduced for further quantitative calculations.

### Example 6: Analysis of mixed sample and internal standard by time-resolved deconvolution MS enables IgG concentration determination with high accuracy

The separation procedure based on time-resolved deconvolution MS as described in Example 5 could be applied to quantitate individual antibodies in a complex antibody mixture. Here, we describe how the antibody sample concentrations can be accurately determined by measuring MS peak volumes/intensities of the antibody samples containing designated IgG-GlcNAc₂ internal standards (IS).

A mixture of antibodies was generated by mixing IgG1-CD19-21D4-E345K, IgG1-CD22-huRFB4, IgG1-7D8, IgG1-CD37-37-3 and IgG1-CD52-Campath-E345K as described in Example 5 with defined antibody concentrations to be used as external calibration standards. A portion of the mixture was treated with EndoS2 and *Lactobacillus casei* fucosidase (29A) as described in Example 5 to generate IS, and another portion was fully deglycosylated using PNGase F, as described in Example 2 to generate antibody external standards. Next, a dilution series was generated of external standards and spiked with one fixed IS concentration to each standard (i.e. XXX µg/mL for each IgG-GlcNAc₂). The antibody external calibration standards vary from 15 - 80 µg/mL per individual antibody. Of each calibration standard, 50 µL was pipetted into Q-sert vials (Sigma). Data were acquired and subsequently processed by

TRD MS as described in Example 2 and 5.
The MS peak volumes/intensities of the calibration standards containing deglycosylated IgG1-CD19-21D4-E345K, IgG1-CD22-huRFB4, IgG1-7D8, IgG1-CD37-37-3 and IgG1-CD52-Campath-E345K and the reference IS IgG-GlcNAc₂ variant were measured by LC-MS and data processed by time-resolved deconvolution.

Calibration curves were generated by first calculating the ratio of the MS peak intensities of the deglycosylated antibodies over the MS peak volume/intensities of the IS (Ext st. /IS). Figure 6A-E shows excellent linearity when plotting the obtained ratios (Y-axis) against the individual standard antibody concentrations. For each generated antibody standard curve, a coefficient of determination (R²) of 0.99 was found, indicating the suitability of the IgG-GlcNAc₂ IS to obtain calibration curves. Figure 6F-J show the antibody sample concentrations calculated for each antibody as deduced from the calibration curve, versus the expected antibody concentration, indicating high accuracy of the measurements.

### Example 7: In-process IgG concentration determination by Time Resolved Deconvolution MS.

By integrating the methods described in Example 5 into an in-process workflow, IgG quantification can be accelerated. To this end, test samples comprising cell culture supernatants are deglycosylated by PNGase F between 2 and 24 hours (using a method similar to that described in Example 2). Subsequently, deglycosylated cell supernatant samples are spiked with previously prepared IS antibodies (Example 5), preferably in the range close to the expected sample IgG concentrations. Next, the prepared samples will be injected into the LC-MS system. The LC-MS system comprises an affinity purification trap column. In this example, the affinity trap column contains immobilized protein A that binds the Fc portion of antibodies. Upon injection of the prepared cell culture samples antibodies are captured on the protein A column. Subsequently, all host cell proteins are washed off the column by utilizing a washing buffer. In the next step, the trapped antibodies are eluted in one step from the column by low pH elution buffer and subsequently separated on a reversed phase (RP) analytical column, which is in-line with the mass spectrometer. The acquired MS data will be processed and antibody masses detected and concentration values will be calculated from the signal intensities according to Example 6.

## Claims

1. A method for the quantification of one or more glycoproteins in a sample, wherein each glycoprotein to be quantified comprises one or more Asn-linked glycans, said method comprising the steps of:
a. providing a sample comprising said one or more glycoproteins to be quantified,
b. adding an internal standard to said sample, wherein said internal standard comprises a variant form of each of said one or more glycoproteins to be quantified, wherein said variant form is a form containing only core Asn-linked GlcNAc moieties, each core Asn-linked GlcNAc moiety being an N-acetylglycosamine moiety that is directly attached to an Asn acceptor residue in the polypeptide chain of said variant, and wherein no fucose moieties are linked to said core GlcNAc moieties; and
c. quantifying said one or more glycoproteins by comparison with the internal standard.

2. The method according to claim 1, wherein the method prior to step c. comprises a treatment of the sample with an enzyme, wherein said enzyme is capable of fully removing Asn-linked glycans from said one or more glycoproteins, but is not capable of removing core Asn-linked GlcNAc moieties from said variant form.

3. The method according to claim 2, wherein said enzyme treatment is performed prior to step b.

4. The method according to claim 2, wherein said enzyme treatment is performed after addition of the internal standard in step b.

5. The method according to any one of claims 2 to 4, wherein said enzyme is PNGase F (EC 3.5.1.52).

6. The method according to claim 1, wherein the method prior to step b. comprises treating the sample with an enzyme capable of fully removing Asn-linked glycans from said one or more glycoproteins, optionally followed by removal or deactivation of said enzyme.

7. The method according to any one of the preceding claims, wherein the quantification in step c. is performed using mass spectrometry.

8. The method according to any one of the preceding claims, wherein the quantification in step c. is performed using a combination of mass spectrometry and a non-mass-based separation technique.

9. The method according to any one of the preceding claims, wherein the quantification in step c. is performed using liquid chromatography-mass spectrometry (LC-MS), such as reverse-phase-based LC-MS.

10. The method according to any one of the preceding claims, wherein said method does not comprise a step of proteolytically digesting, or otherwise fragmenting, the amino acid chain of the glycoproteins to be quantified.

11. The method according to any one of the preceding claims, wherein both the one or more glycoprotein(s) to be quantified and the variant form(s) thereof used in the internal standard are used in their full-length forms, i.e. having a full-length amino acid chain, or almost full-length forms, e.g. comprising 50% or more, such as 75% or more, e.g. 90% or more, such as 95% or more of their full-length amino acid sequence.

12. The method according to any one of the preceding claims, wherein the one or more glycoproteins to be quantified are antibodies.

13. The method according to any one of the preceding claims, wherein the method comprises quantification of two or more, such as two, three, four, five or more different antibodies.

14. The method according to claim 13, wherein said two or more antibodies are IgG antibodies, such as full-length IgG antibodies.

15. The method according to any one of the preceding claims, wherein at least one of the one or more glycoproteins to be quantified contains fucose on core Asn-linked GlcNAc moieties.

16. The method according to any one of the preceding claims, wherein the sample is a cell culture sample.

17. The method according to any one of the preceding claims, wherein the sample comprises purified recombinantly produced glycoproteins.

18. The method according to claim 16 or 17, wherein the steps b. and c. of the method are performed in an automated manner, preferably wherein steps a., b. and c. are performed in an automated manner.

19. The method according to any one of claims 1 to 15, wherein the sample is a blood sample, plasma sample or serum sample.

20. A process for monitoring production of glycoproteins in a cell culture, said process comprising culturing host cells producing said glycoproteins and performing the method according to claim 16 or 18.

21. A process for the quality control of purified recombinantly produced glycoproteins, said process comprising performing the method according to claim 17 or 18.

22. Use of a composition comprising a known quantity of a variant form of a glycoprotein, wherein said variant form is a form containing only core Asn-linked GlcNAc moieties, each core Asn-linked GlcNAc moiety being an N-acetylglycosamine moiety that is directly attached to an Asn acceptor residue in the polypeptide chain of said variant, and wherein no fucose moieties are linked to said core GlcNAc moieties as an internal standard for quantification of said glycoprotein in a sample.

23. A method for the quantification of one or more glycoproteins in a sample, wherein each glycoprotein to be quantified comprises one or more Asn-linked glycans, said method comprising the steps of:
a. preparing an internal standard by treating said one or more glycoproteins with one or more enzymes to obtain a variant form of each of said one or more glycoproteins, wherein said variant form is a form containing only core Asn-linked GlcNAc moieties, each core Asn-linked GlcNAc moiety being an N-acetylglycosamine moiety that is directly attached to an Asn acceptor residue in the polypeptide chain of said variant, and wherein no fucose moieties are linked to said core GlcNAc moieties,
b. providing a sample comprising said one or more glycoproteins to be quantified,
c. adding said internal standard to said sample, and
d. quantifying said one or more glycoproteins by comparison with the internal standard.

24. The method according to claim 23, wherein said treatment with one or more enzymes in step a. comprises treatment with an endo-β-N-acetylglucosaminidase, such as EndoS2 or EndoS.

25. The method according to any of claims 23 to 24, wherein said treatment with one or more enzymes in step a. comprises treatment with an endo-β-N-acetylglucosaminidase, such as EndoS2 or EndoS and a fucosidase, capable of hydrolyzing alpha-1,6-linkage from an Asn-linked fucose-alpha-1,6-GlcNAc moiety.

26. The method according to any one of claims 23 to 25, comprising one or more of the further features of claim 2 to 19.

## Patentansprüche

1. Verfahren zur Quantifizierung eines oder mehrerer Glykoproteine in einer Probe, wobei jedes zu quantifizierende Glykoprotein ein oder mehrere Asn-verknüpfte Glykane umfasst, wobei das Verfahren die folgenden Schritte umfasst:
a. Bereitstellen einer Probe, die das eine oder die mehreren zu quantifizierenden Glykoproteine umfasst,
b. Zugeben eines internen Standards zu der Probe, wobei der interne Standard eine Variantenform jedes des einen oder der mehreren zu quantifizierenden Glykoproteine umfasst, wobei die Variantenform eine Form ist, die nur Kern-Asn-verknüpfte GlcNAc-Einheiten enthält, wobei jede Kern-Asn-verknüpfte GlcNAc-Einheit eine N-Acetylglycosamin-Einheit ist, die direkt an einen Asn-Akzeptorrest in der Polypeptidkette der Variante gebunden ist, und wobei keine Fucose-Einheiten mit den Kern-GlcNAc-Einheiten verknüpft sind; und
c. Quantifizieren des einen oder der mehreren Glykoproteine durch Vergleich mit dem internen Standard.

2. Verfahren nach Anspruch 1, wobei das Verfahren vor Schritt c. eine Behandlung der Probe mit einem Enzym umfasst, wobei das Enzym in der Lage ist, Asn-verknüpfte Glykane aus dem einen oder den mehreren Glykoproteinen vollständig zu entfernen, aber nicht in der Lage ist, Kern-Asn-verknüpfte GlcNAc-Einheiten aus der Variantenform zu entfernen.

3. Verfahren nach Anspruch 2, wobei die Enzymbehandlung vor Schritt b. durchgeführt wird.

4. Verfahren nach Anspruch 2, wobei die Enzymbehandlung nach Zugabe des internen Standards in Schritt b. durchgeführt wird.

5. Verfahren nach einem der Ansprüche 2 bis 4, wobei das Enzym PNGase F (EC 3.5.1.52) ist.

6. Verfahren nach Anspruch 1, wobei das Verfahren vor Schritt b. die Behandlung der Probe mit einem Enzym umfasst, das in der Lage ist, Asn-verknüpfte Glykane aus dem einen oder den mehreren Glykoproteinen vollständig zu entfernen, gegebenenfalls gefolgt von der Entfernung oder Deaktivierung des Enzyms.

7. Verfahren nach einem der vorhergehenden Ansprüche, wobei die Quantifizierung in Schritt c. unter Verwendung von Massenspektrometrie durchgeführt wird.

8. Verfahren nach einem der vorhergehenden Ansprüche, wobei die Quantifizierung in Schritt c. unter Verwendung einer Kombination von Massenspektrometrie und einer nicht auf Masse basierenden Trenntechnik durchgeführt wird.

9. Verfahren nach einem der vorhergehenden Ansprüche, wobei die Quantifizierung in Schritt c. unter Verwendung von Flüssigchromatographie-Massenspektrometrie (LC-MS), wie z.B. auf Reverse-Phase basierender LC-MS, durchgeführt wird.

10. Verfahren nach einem der vorhergehenden Ansprüche, wobei das Verfahren keinen Schritt des proteolytischen Verdauens oder anderweitigen Fragmentierens der Aminosäurekette der zu quantifizierenden Glykoproteine umfasst.

11. Verfahren nach einem der vorhergehenden Ansprüche, wobei sowohl das eine oder die mehreren zu quantifizierenden Glykoproteine als auch die Variantenform(en) davon, die in dem internen Standard verwendet werden, in ihren Volllängenformen, d.h. mit einer Volllängen-Aminosäurekette, oder fast Volllängenformen, die z.B. 50% oder mehr, wie z.B. 75% oder mehr, z.B. 90% oder mehr, wie z.B. 95% oder mehr, ihrer Volllängen-Aminosäuresequenz umfassen, verwendet werden.

12. Verfahren nach einem der vorhergehenden Ansprüche, wobei das eine oder die mehreren zu quantifizierenden Glykoproteine Antikörper sind.

13. Verfahren nach einem der vorhergehenden Ansprüche, wobei das Verfahren die Quantifizierung von zwei oder mehr, wie z.B. zwei, drei, vier, fünf oder mehr, verschiedenen Antikörpern umfasst.

14. Verfahren nach Anspruch 13, wobei die zwei oder mehr Antikörper IgG-Antikörper, wie z.B. Volllängen-IgG-Antikörper, sind.

15. Verfahren nach einem der vorhergehenden Ansprüche, wobei mindestens eines des einen oder der mehreren zu quantifizierenden Glykoproteine Fucose an Kern-Asnverknüpften GlcNAc-Einheiten enthält.

16. Verfahren nach einem der vorhergehenden Ansprüche, wobei die Probe eine Zellkulturprobe ist.

17. Verfahren nach einem der vorhergehenden Ansprüche, wobei die Probe gereinigte rekombinant hergestellte Glykoproteine umfasst.

18. Verfahren nach Anspruch 16 oder 17, wobei die Schritte b. und c. des Verfahrens auf automatisierte Weise durchgeführt werden, vorzugsweise wobei die Schritte a., b. und c. auf automatisierte Weise durchgeführt werden.

19. Verfahren nach einem der Ansprüche 1 bis 15, wobei die Probe eine Blutprobe, Plasmaprobe oder Serumprobe ist.

20. Verfahren zum Überwachen der Produktion von Glykoproteinen in einer Zellkultur, wobei das Verfahren das Kultivieren von Wirtszellen, die die Glykoproteine produzieren, und das Durchführen des Verfahrens nach Anspruch 16 oder 18 umfasst.

21. Verfahren zur Qualitätskontrolle von gereinigten rekombinant hergestellten Glykoproteinen, wobei das Verfahren das Durchführen des Verfahrens nach Anspruch 17 oder 18 umfasst.

22. Verwendung einer Zusammensetzung, die eine bekannte Menge einer Variantenform eines Glykoproteins umfasst, wobei die Variantenform eine Form ist, die nur Kern-Asn-verknüpfte GlcNAc-Einheiten enthält, wobei jede Kern-Asn-verknüpfte GlcNAc-Einheit eine N-Acetylglycosamin-Einheit ist, die direkt an einen Asn-Akzeptorrest in der Polypeptidkette der Variante gebunden ist, und wobei keine Fucose-Einheiten mit den Kern-GlcNAc-Einheiten verknüpft sind, als ein interner Standard zur Quantifizierung des Glykoproteins in einer Probe.

23. Verfahren zur Quantifizierung eines oder mehrerer Glykoproteine in einer Probe, wobei jedes zu quantifizierende Glykoprotein ein oder mehrere Asn-verknüpfte Glykane umfasst, wobei das Verfahren die folgenden Schritte umfasst:
a. Herstellen eines internen Standards durch Behandeln des einen oder der mehreren Glykoproteine mit einem oder mehreren Enzymen, um eine Variantenform jedes des einen oder der mehreren Glykoproteine zu erhalten, wobei die Variantenform eine Form ist, die nur Kern-Asn-verknüpfte GlcNAc-Einheiten enthält, wobei jede Kern-Asn-verknüpfte GlcNAc-Einheit eine N-Acetylglycosamin-Einheit ist, die direkt an einen Asn-Akzeptorrest in der Polypeptidkette der Variante gebunden ist, und wobei keine Fucose-Einheiten mit den Kern-GlcNAc-Einheiten verknüpft sind,
b. Bereitstellen einer Probe, die das eine oder die mehreren zu quantifizierenden Glykoproteine umfasst,
c. Zugeben des internen Standards zu der Probe, und
d. Quantifizieren des einen oder der mehreren Glykoproteine durch Vergleich mit dem internen Standard.

24. Verfahren nach Anspruch 23, wobei die Behandlung mit einem oder mehreren Enzymen in Schritt a. eine Behandlung mit einer Endo-β-N-acetylglucosaminidase, wie EndoS2 oder EndoS, umfasst.

25. Verfahren nach einem der Ansprüche 23 bis 24, wobei die Behandlung mit einem oder mehreren Enzymen in Schritt a. eine Behandlung mit einer Endo-β-N-acetylglucosaminidase, wie EndoS2 oder EndoS, und einer Fucosidase, die in der Lage ist, eine alpha-1,6-Verknüpfung aus einer Asnverknüpften Fucose-alpha-1,6-GlcNAc-Einheit zu hydrolysieren, umfasst.

26. Verfahren nach einem der Ansprüche 23 bis 25, umfassend eines oder mehrere der weiteren Merkmale von Anspruch 2 bis 19.

## Revendications

1. Procédé de quantification d'une ou plusieurs glycoprotéine(s) dans un échantillon, dans lequel chaque glycoprotéine à quantifier comporte un ou plusieurs glycane(s) attaché(s) à Asn, et lequel procédé comporte les étapes suivantes :
a) prendre un échantillon comprenant ladite ou lesdites glycoprotéine(s),
b) ajouter un étalon interne audit échantillon, lequel étalon interne comprend une forme variante de ladite ou de chacune desdites glycoprotéine(s) à quantifier, laquelle forme variante est une forme qui ne contient que des entités GlcNAc de coeur attachées à Asn, étant entendu que chaque entité GlcNAc de coeur attachée à Asn est une entité N-acétyl-glycosamine directement attachée à un résidu accepteur Asn de la chaîne polypeptidique de ladite variante, et dans laquelle il n'y a pas d'entités fucose attachées auxdites entités GlcNAc de coeur,
c) et quantifier ladite ou lesdites glycoprotéine(s) par comparaison avec l'étalon interne.

2. Procédé conforme à la revendication 1, lequel procédé comporte, avant l'étape c), un traitement de l'échantillon avec une enzyme, laquelle enzyme est capable de séparer complètement de ladite ou desdites glycoprotéine(s) les glycanes attachés à Asn, mais n'est pas capable de séparer de ladite forme variante les entités GlcNAc de coeur attachées à Asn.

3. Procédé conforme à la revendication 2, dans lequel ledit traitement avec une enzyme est réalisé avant l'étape b).

4. Procédé conforme à la revendication 2, dans lequel ledit traitement avec une enzyme est réalisé après l'addition de l'étalon interne qui a lieu dans l'étape b).

5. Procédé conforme à l'une des revendications 2 à 4, dans lequel ladite enzyme est la PNGase F (EC 3.5.1.52).

6. Procédé conforme à la revendication 1, lequel procédé comporte, avant l'étape b), un traitement de l'échantillon avec une enzyme capable de séparer complètement de ladite ou desdites glycoprotéine(s) les glycanes attachés à Asn, traitement suivi, en option, de l'élimination ou de la désactivation de ladite enzyme.

7. Procédé conforme à l'une des revendications précédentes, dans lequel, dans l'étape c), la quantification est réalisée par spectrométrie de masse.

8. Procédé conforme à l'une des revendications précédentes, dans lequel, dans l'étape c), la quantification est réalisée au moyen d'une combinaison de la spectrométrie de masse et d'une technique de séparation qui ne repose pas sur la masse.

9. Procédé conforme à l'une des revendications précédentes, dans lequel, dans l'étape c), la quantification est réalisée par chromatographie en phase liquide couplée à la spectrométrie de masse (CL-SM), comme la CL à polarité de phases inversée couplée à la SM.

10. Procédé conforme à l'une des revendications précédentes, lequel procédé ne comporte pas d'étape de digestion protéolytique, ou de fragmentation d'une autre manière, de la chaîne d'acides aminés des glycoprotéines à quantifier.

11. Procédé conforme à l'une des revendications précédentes, dans lequel aussi bien la ou les glycoprotéine(s) à quantifier que sa ou leurs forme(s) variante(s) utilisée(s) dans l'étalon interne sont utilisées sous leurs formes complètes, c'est-à-dire possédant une chaîne d'acides aminés complète, ou presque complètes, par exemple comportant 50 % ou plus, comme 75 % ou plus, par exemple 90 % ou plus, comme 95 % ou plus, de leur séquence complète d'acides aminés.

12. Procédé conforme à l'une des revendications précédentes, dans lequel la ou les glycoprotéine(s) à quantifier est (sont) un (des) anticorps.

13. Procédé conforme à l'une des revendications précédentes, lequel procédé comporte la quantification de deux anticorps ou plus, comme de deux, trois, quatre ou cinq anticorps différents ou plus.

14. Procédé conforme à la revendication 13, dans lequel les deux anticorps ou plus sont des anticorps IgG, comme des anticorps IgG complets.

15. Procédé conforme à l'une des revendications précédentes, dans lequel la glycoprotéine ou au moins l'une des glycoprotéine(s) à quantifier contient du fucose sur des entités GlcNAc de coeur attachées à Asn.

16. Procédé conforme à l'une des revendications précédentes, dans lequel l'échantillon est une échantillon de culture cellulaire.

17. Procédé conforme à l'une des revendications précédentes, dans lequel l'échantillon comprend des glycoprotéines produites par recombinaison et purifiées.

18. Procédé conforme à la revendication 16 ou 17, dans lequel les étapes b) et c) du procédé sont réalisées de manière automatisée, et de préférence, dans lequel les étapes a), b) et c) du procédé sont réalisées de manière automatisée.

19. Procédé conforme à l'une des revendications 1 à 15, dans lequel l'échantillon est un échantillon de sang, un échantillon de plasma ou un échantillon de sérum.

20. Procédé visant à surveiller la production de glycoprotéines dans une culture cellulaire, lequel procédé comporte le fait de cultiver des cellules hôtes produisant lesdites glycoprotéines et le fait d'exécuter un procédé conforme à la revendication 16 ou 18.

21. Procédé visant à maîtriser la qualité de glycoprotéines produites par recombinaison et purifiées, lequel procédé comporte le fait d'exécuter un procédé conforme à la revendication 17 ou 18.

22. Utilisation d'une composition comprenant une quantité connue d'une forme variante d'une glycoprotéine, laquelle forme variante est une forme qui ne contient que des entités GlcNAc de coeur attachées à Asn, étant entendu que chaque entité GlcNAc de coeur attachée à Asn est une entité N-acétyl-glycosamine directement attachée à un résidu accepteur Asn de la chaîne polypeptidique de ladite variante, et dans laquelle il n'y a pas d'entités fucose attachées auxdites entités GlcNAc de coeur, en tant qu'étalon interne pour la quantification de ladite glycoprotéine dans un échantillon.

23. Procédé de quantification d'une ou plusieurs glycoprotéine(s) dans un échantillon, dans lequel chaque glycoprotéine à quantifier comporte un ou plusieurs glycane(s) attaché(s) à Asn, et lequel procédé comporte les étapes suivantes :
a) préparer un étalon interne en traitant ladite ou lesdites glycoprotéine(s) avec une ou plusieurs enzymes pour obtenir une forme variante de ladite ou de chacune desdites glycoprotéine(s), laquelle forme variante est une forme qui ne contient que des entités GlcNAc de coeur attachées à Asn, étant entendu que chaque entité GlcNAc de coeur attachée à Asn est une entité N-acétyl-glycosamine directement attachée à un résidu accepteur Asn de la chaîne polypeptidique de ladite variante, et dans laquelle il n'y a pas d'entités fucose attachées auxdites entités GlcNAc de coeur,
b) prendre un échantillon comprenant ladite ou lesdites glycoprotéine(s) à quantifier,
c) ajouter ledit étalon interne audit échantillon,
d) et quantifier ladite ou lesdites glycoprotéine(s) par comparaison avec l'étalon interne.

24. Procédé conforme à la revendication 23, dans lequel, dans l'étape a), ledit traitement avec une ou plusieurs enzyme(s) comprend un traitement avec une endo-β-N-acétyl-glucosaminidase, telle que EndoS2 ou EndoS.

25. Procédé conforme à l'une des revendications 23 et 24, dans lequel, dans l'étape a), ledit traitement avec une ou plusieurs enzyme(s) comprend un traitement avec une endo-β-N-acétyl-glucosaminidase, telle que EndoS2 ou EndoS, et avec une fucosidase, capable d'hydrolyser la liaison alpha-1,6 d'une entité fucose-alpha-1,6-GIcNAc attachée à Asn.

26. Procédé conforme à l'une des revendications 23 à 25, qui comporte une ou plusieurs caractéristique(s) supplémentaire(s) indiquée(s) dans les revendications 2 à 19.
